# EUROPÄISCHE PATENTSCHRIFT

(11) **EP 1 244 462 B1**
(45) Veröffentlichungstag und Bekanntmachung des Hinweises auf die Patenterteilung: **29.09.2004**
(21) Anmeldenummer: 00987414.0
(22) Anmeldetag: 18.12.2000
(51) Int. Cl.: A61K 35/00, A61P 35/00

(54) **PHARMAZEUTISCHE ZUSAMMENSETZUNG AUS SPINNENGIFTEN SOWIE DEREN HERSTELLUNG UND VERWENDUNG ZUR BEHANDLUNG VON TUMORERKRANKUNGEN**
PHARMACEUTICAL COMPOSITION COMPRISED OF SPIDER VENOMS, THE PRODUCTION THEREOF, AND ITS USE FOR TREATING TUMOR DISEASES
PREPARATION PHARMACEUTIQUE A BASE DE VENIN D'ARAIGNEE, SA PREPARATION ET SON UTILISATION POUR TRAITER DES AFFECTIONS TUMORALES

(30) Priorität: 17.12.1999 DE 19961141
(43) Veröffentlichungstag der Anmeldung: 02.10.2002
(73) Patentinhaber: TOXIMED GmbH, 80331 München (DE)
(72) Erfinder: WEICKMANN, Dirk, 80339 München (DE)
(74) Vertreter: Behnisch, Werner, Dr.
(86) Internationale Anmeldenummer: PCT/EP2000/012902
(87) Internationale Veröffentlichungsnummer: WO 2001/043754

(56) Entgegenhaltungen:
- WO-A-95/19989
- US-A- 4 877 741
- BURDA R. ET AL: "[Cell destructive toxic components of spiders of the genus Sicarius for tumor therapy]. ZELLZERSTORENDE GIFTKOMPONENTEN VON SPINNEN DER GATTUNG SICARIUS BEI DER THERAPIE VON TUMORERKRANKUNGEN." ARZTEZEITSCHRIFT FUR NATURHEILVERFAHREN, (2000) 41/3 (172-177). , XP000994986
- SUAREZ G ET AL: "EFFECT OF EXTRACTS OF THE VENOM GLAND OF THE RECLUSE SPIDER LOXOSCELES-LAETA ON HUMAN CELLS IN CULTURE." TOXICON, (1976) 14 (4), 335-337. , XP000994884
- WRIGHT R P ET AL: "HYALURONIDASE AND ESTERASE ACTIVITIES OF THE VENOM OF THE POISONOUS BROWN RECLUSE SPIDER." ARCH BIOCHEM BIOPHYS, (1973) 159 (1), 415 - 426. , XP001000331
- GEREN C R ET AL: "ISOLATION AND CHARACTERIZATION OF TOXINS FROM BROWN RECLUSE SPIDER VENOM LOXOSCELES-RECLUSA." ARCH BIOCHEM BIOPHYS, (1976) 174 (1), 90 - 99. , XP001000332
- MERCHANT, MICHAEL (1) ET AL: "Observation of phospholipase activity in the venom of the brown recluse spider (Loxosceles reclusa) by phosphorus-31 NMR." FASEB JOURNAL, (1995) VOL. 9, NO. 6, PP. A1313. MEETING INFO.: ANNUAL MEETING OF THE AMERICAN SOCIETY FOR BIOCHEMISTRY AND MOLECULAR BIOLOGY SAN FRANCISCO, CALIFORNIA, USA MAY 21-25, 1995 , XP000995008

## Beschreibung

Die vorliegende Erfindung betrifft pharmazeutische Zusammensetzungen, enthaltend zumindest ein Peptidtoxin sowie zumindest eine hierzu antagonistisch wirkende Substanz und/oder eine Durchdringungssubstanz in einer pharmazeutisch wirksamen Menge, wobei zumindest das Peptidtoxin, und wahlweise die antagonistisch wirkende Substanz und/oder die Durchdringungssubstanz, aus dem Gift von Spinnen der Familie der Sicariidae stammen, sowie die Herstellung und Verwendung der pharmazeutischen Zusammensetzungen.

### Stand der Technik

Bei lokal manifestierten Tumoren ist die möglichst vollständige operative Entfernung des Tumors die derzeit gängigste Therapieform. Der Tumor wird im Vorfeld der Operation über bildlich darstellende Verfahren lokalisiert und über einen öffnenden Eingriff manuell herausgeschnitten. Dabei ist der Kontakt der Operationsfläche mit Luft nicht zu verhindern. Aus der Literatur (Stegner H.-E. (1986): Histopathologie der Mammatumoren. Enke Verlag, Stuttgart; Garbe C., Dummer R., Kaufmann R. und Tielgen W. (1997): Dermatologische Onkologie. Springer Verlag, Berlin (siehe auch Errata)) ist bekannt, dass durch den Luftkontakt mit einer 93%-igen Metastasierungsrate des Primärtumorgewebes zu rechnen ist.

Weitere Therapieformen zur Tumorbehandlung sind die Chemotherapie, die Bestrahlung, Antikörpertherapie, Behandlung mit Zytokinen, Hyperthermie oder die Sauerstofftherapie.

Bei der Chemotherapie von Tumoren werden üblicherweise Zellgifte eingesetzt, um im ganzen Körper verbreitete Tumoren und nach chirurgischer Behandlung lokaler Tumoren verbliebene Tumorzellen zu behandeln (Römpp, Chemielexikon, 9. Auflage, Band 1, 1989, S. 680). Substanzen, die in der Chemotherapie verwendet werden, sind beispielsweise alkylierende Substanzen, Antimetabolite, Alkaloide, Antibiotika und Hormone (Römpp Lexikon, Biotechnologie und Gentechnik, 2. Auflage, 1999, S. 153). Als alkylierende Verbindungen sind beispielweise Cisplatin, Nitrosoharnstoffverbindungen oder Thiotepa bekannt. Weiterhin werden Folsäureantagonisten, z.B. Aminopterin, Pyrimidinanaloga, wie Fluorouracile eingesetzt. Als Antibiotika mit hemmender Wirkung auf die DNA-abhängige RNA-Polymerase seien Bleomycin, Doxorubicin oder Mitomycin C genannt. Auch Enzyme, z.B L-Asparaginase, werden in der Chemotherapie eingesetzt.

Nachteile der Chemotherapie sind, daß die Chemotherapeutika nur schwer gezielt einzusetzen sind und, daß diese Zytostatika äußerst agressive Zellgifte sind, die neben Tumorgewebe auch im großen Maße gesundes Gewebe, einschließlich Leber- und Nierenzellen schädigen. Die auftretenden Nebenwirkungen, wie z.B. Haarausfall, Schwindel, Erbrechen, Magen-Darmblutungen, Kreislaufbeschwerden u.a. sind wegen der systemischen Ausbreitung der Zytostatika nur schwer abzuwägen (Deutsches Krebsforschungszentrum DKFZ Heidelberg - Focus 19/1995). Diese zahlreichen, gefährlichen und unerwünschten Nebenwirkungen erklären sich vor allem aus der Regenerationshemmung schnell proliferierender Gewebe und betreffen insbesonders das blutbildende System, die Epithelien der Schleimhäute und der Gonaden sowie die Haut und Hautanhangsgebilde. Unter den lebensbedrohlichen Komplikationen stehen Infektionen an erster Stelle gefolgt von Blutungen (Pschyrembel - Klinisches Wörterbuch 256. Auflage 1990, Seite 1866).

Die Bestrahlung erfolgt mit ionisierender Strahlung, üblicherweise verwendet man Elektronen-, Gamma-, Neutronen- oder Röntgenstrahlen (Zetkin/Schaldach: Lexikon der Medizin, 16. Auflage 1999, Seite 1922/1923 Ullstein Medical). Nachteil der Bestrahlung ist, dass ähnlich wie bei der Chemotherapie eine räumliche Begrenzung nicht möglich ist. Durch die Strahlungshärte werden auch gesunde Zellen nachhaltig geschädigt, vor allem die DNA. Da Krebszellen sich meist schneller als gesunde Zellen teilen, werden die Krebszellen unter normalen Umständen bei der Strahlentherapie als erstes abgetötet. Allerdings besteht dabei die Gefahr eines Strahlenulkus (Pschyrembel - Klinisches Wörterbuch 256, Auflage 1990, Seite 1602).

Es ist daher eine Aufgabe dieser Erfindung, verbesserte Mittel und Verfahren bereitzustellen, die für die Tumortherapie und/oder als Begleittherapie, z.B. bei der chirurgischen/operativen Behandlung von Tumorerkrankungen, eingesetzt werden können und die genannten Nachteile des Stands der Technik nicht aufweisen.

Dies wird erfindungsgemäß durch eine pharmazeutische Zusammensetzung erreicht, enthaltend in einer pharmazeutisch wirksamen Menge:
a) zumindest ein Peptidtoxin, sowie
b) zumindest eine hierzu antagonistisch wirkende Substanz und/oder zumindest eine Durchdringungssubstanz,
wobei zumindest das Peptidtoxin aus dem Gift von Spinnen der Familie der Sicariidae stammt und wahlweise die hierzu antagonistisch wirkende Substanz und/oder die Durchdringungssubstanz aus dem Gift von Spinnen der Familie der Sicariidae stammt.

Überdies können vorteilhafterweise ein oder mehrere weitere Substanzen aus dem Gift von Spinnen der Familie der Sicariidae in den erfindungsgemäßen pharmazeutischen Zusammensetzungen enthalten sein. In einer weiteren Ausführungsform können bevorzugt weitere Substanzen aus anderen Gifte enthaltenden Organismen enthalten sein.

Unter den Spinnen der Familie der Sicariidae sind die Gattungen Sicarius, Loxosceles, Scytodes und Drymusa bevorzugt.

Bevorzugt sind pharmazeutische Zusammensetzungen, bei denen das Peptidtoxin sowie die antagonistisch wirkende Substanz und/oder die Durchdringungssubstanz aus dem Gift von Sicarius-, Loxosceles-, Scytodes- und Drymusa-Spinnenarten stammen.

Bevorzugt sind weiterhin pharmazeutische Zusammensetzungen, bei denen das Peptidtoxin sowie die antagonistisch wirkende Substanz und/oder die Durchdringungssubstanz aus dem Gift der Sicarius-Spinnenarten Sicarius oweni, Sicarius testaceus, Sicarius hahni und Sicarius albospinosus, der Loxosceles-Spinnenarten Loxosceles reclusa, Loxosceles rufipes und Loxosceles laeta, und/oder der Scytodes-Spinnenarten Scytodes thoracica, Scytodes rufa und Scytodes longipes stammen. Dies hat den Vorteil, daß dadurch das von der Natur gegebene Zusammenspiel von Peptidtoxinen und dazu antagonistisch wirkenden Substanzen eines einzigen Organismus ausgenutzt werden kann.

Gemäß der vorliegenden Erfindung können die antagonistisch wirkende Substanz und/oder die Durchdringungssubstanz aber auch aus einem anderen Organismus stammen oder synthetisch oder gentechnisch hergestellt worden sein oder es kann ein weiteres Peptiotoxin aus einem anderen Organismus enthalten sein: Beispielsweise kann als weiteres Peptidtoxin das Schlangengift Captopril enthalten sein oder die antagonistisch wirkende Substanz kann eine Hyaluronidase aus Cobragiften sein.

Erfindungsgemäß hat das eingesetzte Peptidtoxin bevorzugt eine zellzerstörende Wirkung.

Die antagonistisch wirkende Substanz und/oder die Durchdringungssubstanz ist bevorzugt eine Phospholipase oder eine Hyaluronidase oder eine Kombination beider Substanzen. Auch andere Substanzen als Phospholipasen oder Hyaluronidasen, die antagonistisch auf das Peptidtoxin und/oder als Durchdringungssubstanz wirken, sind erfindungsgemäß umfaßt. Weiterhin ist bevorzugt, daß die antagonistisch wirkende Substanz eine Mischung aus im Gift von Spinnen der in dieser Erfindung genannten Arten vorhandenen Phospholipasen und Hyaluronidasen ist. In einer weiteren Ausführungsform ist die antagonistisch wirkende Substanz und/oder die Durchdringungssubstanz eine Phospholipase und/oder eine Hyaluronidase aus einem anderen Organismus als Spinnen der Familie der Sicariidae, z.B. andere Spinnenfamilien oder Schlangen. Bevorzugt sind Hyaluronidasen aus Schlangengiften, bevorzugt aus Cobragiften, oder Phospholipasen aus Actrataspis bibronii, Bitis arietans oder Vipera aspis zinnikeri enthalten. Die Durchdringungssubstanz ist bevorzugt eine Phospholipase.

Bevorzugt werden das Peptidtoxin und die hierzu antagonistisch wirksame Substanz und/oder die Durchdringungssubstanz durch ein Fraktionierungsverfahren aus dem Spinnengift erhalten, und es ist weiterhin bevorzugt, daß die pharmazeutische Zusammensetzung ein Peptidtoxin und eine hierzu antagonistisch wirkende Substanz und/oder eine Durchdringungssubstanz enthält, die aus verschiedenen Fraktionen stammen. Dadurch kann die pharmazeutische Zusammensetzung in ihrer Wirkung vorteilhafterweise auf die zu behandelnde Tumorart und/oder -größe abgestimmt werden.

Das Peptidtoxin und die hierzu antagonistisch wirkende Substanz und/oder die Durchdringungssubstanz können durch an sich bekannte Fraktionierungsverfahren zur Auftrennung von Proteinen aus dem Spinnengift-Rohgemisch (Spinnengiftcocktail) erhalten werden. Bevorzugt ist, daß das Peptidtoxin und die hierzu antagonistisch wirkende Substanz durch Gelchromatographie, HPLC, Affinitätschromatographie und/oder lonenaustauschchromatographie erhalten werden. Die antagonistisch wirkende Substanz und die Durchdringungssubstanz können auf gleiche Weise auch aus anderen Organismen gewonnen werden.

Bevorzugt ist außerdem, daß das Peptidtoxin und die hierzu antagonistisch wirkende Substanz und/oder die Durchdringungssubstanz in einer solchen Menge in der pharmazeutischen Zusammensetzung vorliegen, daß eine bezüglich Tumorzellen zerstörende Wirkung des Peptidtoxins und der hierzu antagonistisch wirkenden Substanz und/oder der Durchdringungssubstanz vorliegt. Das Verhältnis bzw. die Menge des Peptidtoxins und der hierzu antagonistisch wirkenden Substanz wird bevorzugt so gewählt, das eine in zeitlicher und/oder räumlicher Richtung im zu behandelnden Gewebe kontrollierte Ausbreitung sichergestellt ist. Weiterhin wird die Menge so gewählt, das das Peptidtoxin keine oder nur eine geringe toxische Nebenwirkung im zu behandelnden Patienten ausübt. Die Mengen sind selbstverständlich auch auf den zu behandelnden Tumor und auf den zu behandelnden Patienten abzustimmen. Die geeignete Menge der einzelnen Substanzen und ihr Verhältnis zueinander sind vom Fachmann im Rahmen von Tierversuchen und/oder ethisch vertretbaren Versuchen am Patienten festzulegen. Die Menge der Durchdringungssubstanz wird bevorzugt so gewählt, dass die Durchdringungssubstanz weitgehend alle entarteten Zellen erkennt und in Kombination mit dem Peptidtoxin selektiv die Tumorzellen zerstört, während gesunde Zellen weitgehend erhalten bleiben.

Weiterhin bevorzugt ist eine pharmazeutische Zusammensetzung, bei der die Menge an Peptidtoxin und hierzu antagonistisch wirkender Substanz und/oder der Durchdringungssubstanz so ausgewählt ist, daß eine räumlich und zeitlich kontrollierte Ausbreitung sichergestellt ist.

Die pharmazeutische Zusammensetzung weist bevorzugt eine Menge an Peptidtoxin und antagonistisch wirkender Substanz und/oder Durchdringungssubstanz auf, die in Abhängigkeit von dem zu behandelnden Tumor gewählt ist.

Es ist weiterhin bevorzugt, daß die pharmazeutische Zusammensetzung übliche Träger- und Hilfsstoffe enthält. Bevorzugt ist, daß die pharmazeutische Zusammensetzung weitere Wirkstoffe wie Antibiotika , Antimykotika, Antituberkulotika, Mittel gegen Parasiten, Zytostatika, Aminosäuren, die Wundheilung begünstigende Enzyme und/oder Mitosehemmstoffe enthält. Bevorzugt sind dabei Penicillin/Streptomycin, Polymxin/Gentamycin, Glutamin (5%), Mitopodozid, Vinca rosea - Alkaloide, Bromeiaina oder Bromelains.

Das in der erfindungsgemäßen pharmazeutischen Zubereitung enthaltende Peptidtoxin aus dem Gift von Spinnen der Familie der Sicariidae und die hierzu antagonistisch wirkende Substanz und/oder die Durchdringungssubstanz können durch an sich bekannte Isolationsverfahren gewonnen werden. Ein bevorzugtes Beispiel hierfür ist ein Fraktionierverfahren. Die so gewonnenen und durch Aufreinigungsverfahren in reiner Form erhaltenen Substanzen können dann einer medizinisch-therapeutischen Anwendung zugeführt werden. Ein bevorzugtes Verfahren wird nachfolgend näher beschrieben.

Es ist aber auch möglich, das Peptidtoxin aus dem Gift von Spinnen der Familie der Sicariidae und die hierzu antagonistisch wirkende Substanz und/oder die Durchdringungssubstanz chemisch-synthetisch oder durch gentechnologische Methoden in rekombinanter Form herzustellen. Wie bei chemischen Substanzen üblich, umfaßt die vorliegende Erfindung auch Derivate und Salze der erfindungsgemäß bereitgestellten Substanzen. Beispielsweise kann das Peptidtoxin ein oder mehrere Additionen, Substitutionen und/oder Deletionen von Aminosäuren umfassen, wobei sichergestellt sein muß, daß die erfindungsgemäße medizinische Wirkung beibehalten bleibt.

Die Gewinnung des Peptidtoxins und der hierzu antagonistisch wirkenden Substanz und/oder der Durchdringungssubstanz erfolgt durch in der chemischen Verfahrenstechnik übliche Methoden. Hierzu gehören insbesondere Fraktionierverfahren; es sind aber auch andere Verfahren einsetzbar, beispielsweise immunologische Verfahren, um die gewünschten Substanzen aus dem Gesamtgift-Cocktail "herauszufischen".

Ein bevorzugtes Verfahren zur Herstellung einer erfindungsgemäßen pharmazeutischen Zusammensetzung, enthaltend in einer pharmazeutisch wirksamen Menge zumindest ein Peptidtoxin sowie zumindest eine hierzu antagonistisch wirkende Substanz und/oder zumindest eine Durchdringungssubstanz, wobei das Peptidtoxin aus dem Gift von Spinnen der Familie der Sicariidae stammt und wahlweise die hierzu antagonistisch wirkende Substanz und/oder die Durchdringungssubstanz aus dem Gift von Spinnen der Familie der Sicariidae stammt, weist folgende Schritte auf:
- Gewinnen eines Spinnengift-Rohgemischs durch an sich bekannte Verfahren sowie Fraktionierung der Mischung, um das Peptidtoxin und wahlweise die hierzu antagonistisch wirkende Substanz und/oder die Durchdringungssubstanz und wahlweise weitere Substanzen in möglichst voneinander getrennten Fraktionen zu erhalten;
- Kombination verschiedener Fraktionen des Peptidtoxins mit Fraktionen, die hierzu antagonistisch wirkende Substanzen und/oder Durchdringungssubstanzen enthalten, oder mit aus anderen Organismen stammenden antagonistisch wirkenden Substanzen und/oder Durchdringungssubstanzen, um eine pharmazeutisch wirksame Zusammensetzung zu erhalten.

Das Spinnengift enthält verschiedene Peptidtoxine und verschiedene hierzu antagonistisch wirkende Substanzen und/oder Durchdringungssubstanzen und gegebenenfalls andere, ebenfalls medizinisch-therapeutisch relevante Wirkstoffe. Alle diese Substanzen können in einem bestimmten, vom Fachmann zu bestimmenden Verhältnis, in einer pharmazeutischen Zubereitung therapeutisch eingesetzt werden. Die in den Ausführungsbeispielen gezeigten Versuche betreffen insbesondere die Fraktionen 1 bis 12, wobei auch die nachfolgenden Fraktionen des hier speziell beschriebenen Fraktionierverfahrens therapeutisch wirksame Substanzen enthalten. Es wird darauf hingewiesen, daß das Fraktionierverfahren lediglich beispielhaft eine Möglichkeit zur Gewinnung der Peptidtoxine und der hierzu antagonistisch wirkenden Substanzen aufzeigt. Weitere Ausgestaltungen sind möglich.

Dabei ist bevorzugt, daß das Spinnengift-Rohgemisch aus weiblichen Spinnen der Familie der Sicariidae gewonnen wird. Dies ist vorteilhaft, da weibliche Spinnen der Familie der Sicariidae mehr Gift produzieren als männliche Spezies.

Es ist weiterhin bevorzugt, daß das Spinnengift-Rohgemisch durch manuelles Melken erhalten wird. Dies hat den Vorteil einer besonders schonenden Gewinnung des Spinnengift-Rohgemischs.

Bei dem erfindungsgemäßen Verfahren ist überdies bevorzugt, daß das Spinnengift-Rohgemisch vor der Fraktionierung homogenisiert wird, und es ist weiterhin bevorzugt, daß die Fraktionen vor der Weiterverarbeitung tiefgekühlt und weiter bevorzugt lyophilisiert werden.

Die erfindungsgemäßen pharmazeutischen Zusammensetzungen eignen sich zur Verwendung in der Medizin.

Die pharmazeutischen Zusammensetzungen können erfindungsgemäß bevorzugt zur Behandlung von Tumorerkrankungen verwendet werden, wobei eine unterstützende Behandlung bei Tumoroperationen weiterhin bevorzugt ist.

### Beschreibung

Es gibt derzeit weltweit ca. 35000 Arten von Webspinnen. Mit Ausnahme von etwa 300 Arten sind dies alles aktiv giftige Tiere, die ihr Gift zum Beutefang benötigen. Da Spinnen nur eine sehr kleine Mundöffnung haben, entwickelten sie Enzyme und Gifte die ihre Beutetiere außerhalb des Körpers verdauen, so daß die Spinnen verflüssigte Nahrung einsaugen. Etwa 50 Spinnenarten könen durch ihre Gifte auch dem Menschen gefährlich werden. Trotzdem sind vor allem die Gifte dieser Arten nur oberflächlich oder gar nicht erforscht. Die Hauptbestandteile von Spinnengiften sind:
- Verdauungsenzyme
- Biogene Amine
- Organische Säuren
- Peptide
- Peptidtoxine.

Unter den Peptidtoxinen finden sich folgende Toxingruppen:
- Herzgifte
- Nervengifte
- Blutgifte
- Zellgifte
- Gewebezerstörende Gifte.

Ursprünglich findet durch den Gesamtgiftcocktail von allen aktiv giftigen Tieren durch das Zusammenwirken verschiedener Substanzen generell auch eine Vorverdauung und damit eine gezielte Ursprungszellenveränderung tierischer Zellen statt.

Bei den in dieser Erfindung verwendeten Spinnenarten finden sich im Giftcocktail Substanzen, die zytotoxisch, nekrotisch und apoptotisch wirken (verdauende Wirkung der Gifte). Daneben finden sich auf diese lysierend wirkenden Substanzen u.a. noch antagonistisch wirkende Stoppsubstanzen und/oder Durchdringungssubstanzen.

Da die Spinne noch verwertbare Nahrung (ganze Proteinstrukturen und intakte Aminosäuren) aufnehmen muss, hat sie im Laufe ihrer 350 Millionen Jahre dauernden Evolution ihren hochwirksamen Giftcocktail entwickelt. Bei diesem Giftcocktail wird durch das Zusammenspiel von Peptidtoxinen mit dagegen antagonistisch wirkenden Substanzen die räumliche Ausbreitung des Peptidtoxins, von Zeit- und Konzentrationsfaktoren abhängig, durch spezifisch wirkende Enzyme kontrolliert begrenzt.

Überraschenderweise wurde nun gefunden, daß Bestandteile der Spinnengifte von Spinnen der Familie der Sicariidae zur Behandlung von Tumorerkrankungen verwendet werden können.

Der Gesamtgiftcocktail ist auf Grund seiner in bereits geringen Dosen letalen Wirkung, welche auf die Synergismen und Antagonismen verschiedener in diesem Gemisch enthaltenen Substanzen zurückzuführen ist, nicht pharmazeutisch einsetzbar. Als Abwehrgift von den Spinnen abgegeben, erleidet der Gebissene folgende Symptomatik:

Der Biss wird von über 90 Prozent der Gebissenen nicht wahrgenommen. Nach ca. 90 Minuten findet man um die Bissstelle eine stark nässende, schon durch die Haut mazerierende Lokalnekrose von ca. 3 cm Durchmesser. Nach etwa 2 Stunden bricht örtlich die Bisswunde auf und erste systemische Wirkungen sind feststellbar, wie Kreislaufversagen und/oder Herzarhythmen. Nach weiteren 2-3 Stunden beginnen die lysierenden Substanzen zu wirken. Der Gebissene verspürt starken Harndrang, wobei der Urin schon blutig ist. Schmerzen breiten sich im gesamten Bauchraum aus durch die organo-nekrotische Wirkung des Giftes. Die Leber schafft es nicht, Gift in den von der Spinne abgegebenen Konzentrationen und Kombinationen zu metabolisieren. Hat die Spinne beim Biss sehr viel Gift injiziert, schafft der Körper den Abbau nicht mehr und der Patient verstirbt an Nierenversagen in Folge von akuter Blutvergiftung.

Überraschenderweise können jedoch Kombinationen von im Spinnengift enthaltenen Peptidtoxinen und gegengerichteten (hierzu antagonistisch wirkenden) Enzymen und/oder Durchdringungssubstanzen in entsprechenden Konzentrationen und Mengenverhältnissen, wobei zumindest das Peptidtoxin und wahlweise die hierzu antagonistisch wirkende Substanz und/oder die Durchdringungssubstanz aus dem Gift von Spinnen der Familie der Sicariidae stammt, zur Behandlung von Tumorerkrankungen sowie parallel bzw. unterstützend zu Tumoroperationen eingesetzt werden und (Rest-) Tumorgewebe zerstört werden. Beispielsweise kann erfindungsgemäß die Zerstörung von bei der Operation nicht erfasstem Tumorgewebe sowie die Verhinderung der Lokaltumor-Metastasenbildung im Organismus erreicht werden.

### Wirkungsweise der mit dem Peptidtoxin kombinierten Substanzen:

**Antagonistische Wirkung:** Gewebe kann erfindungsgemäß in vitro in gewünschten, örtlich festgelegten Bereichen, insbesondere tumorzellprädestinierten Bereichen, komplikationsfrei abgetötet werden. Die Funktionsweise basiert hier auf nativen, sich gegenseitig beeinflussenden Wirkweisen der Peptidtoxine und der im Giftcocktail vorhandenen hierzu antagonistisch wirkenden Substanzen. Unter antagonistisch wirkenden Substanzen sind erfindungsgemäß solche Substanzen zu verstehen, die das hiermitkombinierte Peptidtoxin verdauen bzw. zersetzen können. Im Humanzellversuch ergab sich folgendes: Ein Teil der zellzerstörend wirkenden Peptidtoxine breitet sich in der Zellkultur schneller aus als die dieses Toxin verdauenden und somit antagonistisch wirkenden Enzyme. Es lässt sich nun eine quantitative Zusammensetzung einer Kombination aus Peptidtoxin und antagonistisch wirkender Substanz ermitteln, in Abhängigkeit von Qualität und Quantität des zu lysierenden Gewebebereiches, so dass eine kontrollierte räumliche und zeitliche Zerstörung erreicht wird. Bei einer einfachen Molekulargewichtsbestimmung mittels Elektrophorese lassen sich nach Inkontaktbringen in Lösung im Vorher-Nachher-Vergleich die ursprünglich eingesetzten Peptidtoxine nicht mehr nachweisen (die jeweilige Bande fehlt). In dieser Erfindung sind unter antagonistisch wirkenden Substanzen beispielsweise Phospholipasen und Hyaluronidasen aus Spinnen der Familie der Sicariidae zu verstehen, wobei nicht ausgeschlossen ist, daß weitere antagonistisch wirkende Substanzen in dem Spinnengift vorhanden sind, die erfindungsgemäß ebenfalls einsetzbar sind.

**Durchdringung** (Synergistische Wirkung): Die Oberflächenproteinstruktur genetisch defekter Körperzellen bzw. Tumorzellen ist verändert (Lottspeich F., Zorbas H. (1998): Bioanalytik. Spektrum Akademischer Verlag Heidelberg Berlin). Die erfindungsgemäß eingesetzten Phospholipasen und/oder Hyaluronidasen und gegebenenfalls weitere Substanzen können nun diese in ihrer Oberflächenstruktur veränderten Tumorzellen erkennen bzw. selektiv binden und lysieren. Erfindungsgemäß trifft dies insbesondere für Phospholipasen zu. Da der Immunstatus der eingesetzten Phospholipasen tierischer Herkunft alleine häufig recht niedrig ist, wie auch der von humanen Phospholipasen Krebskranker, können erfindungsgemäß Phospholipasen anderer Organismen, z.B. Spinnen oder Schlangen, für den Therapieansatz zur Behandlung von Tumorerkrankungen eingesetzt werden. Experimentell zeigte sich, dass auch körperfremde Phospholipasen aus Spinnen- bzw. Schlangengiften genetisch defekte humane Körperzellen nicht nur erkennen, sondern auch zur Infiltration von nekrotisch bzw. zytotoxisch wirkenden Peptiden, welche an sie gekoppelt sind, geeignet sind. Diese Peptide, in unserem Fall Peptidtoxine aus den Giften der Familie der Sicariidae, wirken, in die Zelle gelangt, zellzerstörend. Vermutlich handelt es sich dabei um Apoptose, da sich nach der Zellzerstörung im Medium (Überstand) Caspase-3, ein wichtiger Apoptosemarker messen lässt.

Unter Durchdringungssubstanzen werden erfindungsgemäß solche Substanzen verstanden, die in Kombination mit dem Peptidtoxin selektiv die Tumorzellen zerstören und gesunde Zellen weitgehend erhalten. Damit wird erfindungsgemäß auch die Fähigkeit von Phospholipasen, Hyaluronidasen oder anderer, im Gift enthaltener Substanzen verstanden, entartete Zellen an ihrer veränderten Oberflächenstruktur erkennen zu können, an diese anzudocken und dabei die Zellwand auflockern zu können, um bspw. an sie gekoppelte Substanzen (bevorzugt zellzerstörend wirkende Peptidtoxine) in die entartete Zelle aktiv zu infiltrieren. Insofern wirken die erfindungsgemäß verwendeten Durchdringungssubstanzen, insbesondere Phosphoöipasen, als Boten- und Hilfstoffe (synergistische Wirkung). Erfindungsgemäß ist mit Durchdringung daher nicht eine permeabilitätssteigernde Wirkung gemeint, unter der in der Literatur zumeist eine verbesserte Ausbreitungstendenz in Geweben verstanden wird.

Die Peptidtoxine mit einem Molekulargewicht von ca. 100 kDa besitzen eine gewebezerstörende Wirkung. Auf Grund ihres hohen Molekulargewichtes und ihrer räumlichen Struktur haben sie in Geweben nur eine Ausbreitungstendenz von etwa 100 Zellschichten pro Pikogramm Substanz.

Gegebenenfalls können weitere im Spinnengift-Rohgemisch enthaltene Substanzen die genannten Wirkungen unterstützen.

Um ungewollte Zellzerstörungen zu verhindern, kann erfindungsgemäß in Abhängigkeit. von Art und Größe des zu behandelnden Tumors ein Abgleich bezüglich absoluter und relativer Mengen der Bestandteile des Peptidtoxin/ Enzymgemisches in vitro an lebenden menschlichen Zellen (gesund und tumorös) des zu therapierenden Gewebetyps erfolgen. Hierbei kommt der Beachtung der Ausbreitungstendenz die größte Bedeutung zu. Diese kann im Vergleich der Tumorgewebsfestigkeit zu dem Tumor umgebenden Gewebe in Vorversuchen abgeklärt werden (s. auch Beispiel 2).

Die Wirkweise von Gesamttiergiftcocktails bzw. einzelner daraus säulenchromatographisch abgetrennter und über das Molekulargewicht charakterisierter Substanzen kann durch Austestung dieser in entsprechenden gesunden und tumorösen humanen Zelllinien erfolgen.

Erfindungsgemäß stammt zumindest das Peptidtoxin und wahlweise die antagonistisch wirkende Substanz und/oder die Durchdringungssubstanz aus dem Gift von Spinnen der Familie der Sicariidae. Bevorzugt sind die Gattungen Sicarius, Loxosceles, Scytodes und/oder Drymusa. Im Bereich der Gattung Sicarius können die Sicarius-Spinnenarten Sicarius oweni, Sicarius testaceus, Sicarius hahni und Sicarius albospinosus besonders bevorzugt verwendet werden. Unter den Spinnen der Gattung der Loxosceles sind erfindungsgemäß die Arten Loxosceles rufescens, Loxosceles reclusa und/oder Loxosceles laeta einsetzbar. Unter den Spinnen der Gattung der Scytodes sind erfindungsgemäß die Arten Scytodes thoracica, Scytodes rufa und/oder Scytodes longipes einsetzbar.

Gemäß der vorliegenden Erfindung stammen die Peptidtoxine bevorzugt aus dem gleichen Organismus wie die hierzu antagonistisch wirkenden Substanzen und/oder die Durchdringungssubstanzen und wahlweise enthaltenen weiteren Wirksubstanzen. Auf diese Weise kann das effektive, von der Natur entwickelte Zusammenspiel dieser Substanzen ausgenutzt werden.

In einer weiteren, ebenfalls bevorzugten Ausführungsform stammen die antagonistisch wirksame Substanz und/oder die Durchdringungssubstanz aus anderen Organismen als Spinnen der Familie der Sicariidae bspw. aus anderen Spinnenfamilien, Schlangen, Skorpionen oder ähnlichem. Hier können häufig größere Mengen der genannten Sunstanzen erhalten werden. Beispiele für solche anderen Organismen sind Cobras, Actrataspis bibronii, Bitis arietans oder Vipera aspis zinnikeri.

Die Herstellung der erfindungsgemäßen pharmazeutischen Zusammensetzungen kann so erfolgen, daß zunächst ein Spinnengift-Rohgemisch durch an sich bekannte Verfahren aus den Spinnen gewonnen wird, und eine Fraktionierung des Spinnengift-Rohgemischs durch ebenfalls an sich bekannte Fraktionierungsverfahren zur Auftrennung von Proteinen vorgenommen wird, um die Peptidtoxine und die hierzu antagonistisch wirkenden Substanzen und/oder die Durchdringungssubstanz in möglichst voneinander getrennter Form beziehungsweise in getrennten Fraktionen zu erhalten. Anschließend können zur Herstellung einer pharmazeutischen Zubereitung verschiedene Fraktionen des Peptidtoxins mit Fraktionen, die hierzu antagonistisch wirkende Substanzen und/oder Durchdringungssubstanzen enthalten kombiniert werden oder einzelne Fraktionen des Peptidtoxins können mit aus anderen Organismen antagonistisch wirkenden Substanzen und/oder Durchdringungssubstanzen kombiniert werden. Bevorzugt können als Peptidtoxine weiterhin auch Schlangengifte, beispielsweise das Grubenottern-Schlangengift Captopril, enthalten sein. Bevorzugt können als antagonistisch wirkende Substanzen auch Hyaluronidasen aus Schlangengiften, beispielsweise aus Cobragiften, eingesetzt werden und/oder als Durchdringungssubstanzen Phospholipasen aus Actrataspis bibronii, Bitis arietans oder Vipera aspis zinnikeri, jeweils kombiniert mit ein oder mehreren Fraktionen des Sicarius-Peptidtoxins.

Es ist erfindungsgemäß auch möglich, zur Herstellung pharmazeutischer Zusammensetzungen, die Fraktionen auch zusätzlich mit weiteren geeigneten Wirkstoffen und/oder mit in der Pharmazie üblichen Träger- und Hilfsstoffen zu kombinieren.

Zur Herstellung der erfindungsgemäßen pharmazeutischen Zusammensetzungen können aus dem Giftcocktail, der über manuelles, Melken der oben genannten Spinnenarten gewonnen werden kann, z.B. über säulenchromatographische Aufreinigung spezifische Giftkomponenten (nekrotisch und zytotoxisch wirkende Peptidtoxine) sowie natürliche hierzu antagonistisch wirkende Substanzen (Stoppsubstanzen) und/oder Durchdringungssubstanzen vom Phospholipase- und Hyaluronidase Typ selektiert werden.

Die Analytik zur Differenzierung der in den Fraktionen enthaltenen Komponenten kann über HPLC-MS-MS (z.B. mit einem Gerät der Firma Perkin-Elmer) erfolgen. Dabei konnte nachgewiesen werden, dass es sich bei den hochmolekularen Substanzen auf Grund ihres MS-MS-analysierten Grundgerüstes um Enzyme vom Phopholipase-und Hyaluronidase-Typ handelt. Neben diesen Enzymen konnten noch Polypeptide gefunden werden, die auf Grund Ihrer Herkunft, ihrer Wirkungsweise und ihrer über die MS-MS-Analyse dargestellten, toxischen Gruppen vom NX-, NHX-, NOX- und SX-Typ, als Peptidtoxine zu klassifizieren sind.

Die erfindungsgemäß für die pharmazeutische Zusammensetzung verwendeten Substanzen können auf natürlichem Wege von Webspinnen der Gattung Sicarius produzierte Gifte, die ursprünglich zum Beutefang und zur Vorverdauung tierischen Proteins entwickelt wurden, enthalten. Diese natürliche Wirkweise kann durch eine funktionserhaltende, schonende Gewinnung des Giftgrundstoffes (z.B. durch manuelles Melken) erhalten werden.

Im Gegensatz zu herkömmlichen Melkweisen von Arthropoden mittels eines elektrischen Verfahrens (Weickmann D. (1991): Haltung und Giftigkeit von Sicariidae. Arachnologischer Anzeiger 16:12-13; Weickmann D, Burda R. (1994): Electrophoresis of scorpion venoms. Electrophoresis Forum 1994, Abstracts, Technische Universität München, Okt. 24-26), bei dem den Tieren das Gift über einen elektrischen Impuls, der bei den Tieren die Kontraktion der Giftdrüsen auslöst, entzogen wird (die Tiere sind hierbei bevorzugt unterkühlt), wird gemäß der vorliegenden Erfindung der Giftcocktail über ein manuelles Verfahren, bei dem die Tiere über die Ausnutzung ihres natürlichen Abwehrverhaltens zur Abgabe ihres Giftes stimuliert werden, erhalten.

Gemäß einer Ausführungsform der Erfindung ist eine manuelle Melkweise der Spinnen vorgesehen. Dadurch werden echte, unverfälschte native Gifte erhalten, während im Gegensatz dazu zum Beispiel bei der elektrischen Melkweise durch Elektronenfluss umstrukturierte Substanzen bzw. Moleküle erhalten werden, die in ihrer Wirkweise geändert sein können, oder auch Substanzen in den Giften vorhanden sein können, die das Tier sonst nicht abgeben würde. Diese Substanzen können, müssen aber nicht zwingend, die Effizienz der im Giftcocktail enthaltenen medizinisch wirksamen Verbindungen negativ beeinflussen. Standardmäßig kann eine Analyse und/oder Qualitätskontrolle des Rohgiftgemischs über elektrophoretische Verfahren erfolgen.

Die folgenden Beispiele zeigen vorteilhafte Ausführungsformen der Erfindung, sollen den Schutzumfang der Erfindung jedoch nicht beschränken.

In den Beispielen und der Beschreibung wird auf die folgenden Figuren Bezug genommen:
Figur 1 zeigt eine SDS-PAGE-Elektrophorese (5 cm Sammelgel, 15 cm Trenngel, Molekulargewichtsbereich von 10 bis 200 kDa) von Gesamtgiftcocktails aus Sicarius testaceus und Sicarius sp. Argentinien. Spuren 1 bis 3 sind Molekulargewichtsstandards, Spuren 4 und 5 sind Gesamtgiftcocktails aus Sicarius testaceus und Spur 6 ist ein Gesamtgiftcocktail von Sicarious sp. Argentinien.
Figur 2 zeigt die Wirkung eines Sicarius-Gesamtgiftcocktails auf eine Mischkultur von gesunden Hautzellen mit Melanomzellen.
Figur 3 zeigt eine mikroskopische Aufnahme zur Wirkung einer erfindungsgemäßen Zusammensetzung (Sic.Tox.3 + Sic.Enz.1) auf in einer Brustgewebezellkultur befindliche Mamma-Carcinom-Zellen, 48 Stunden nach Injektion.
Figur 4 zeigt den gleich Ausschnitt wie Figur 3, jedoch 72 Stunden nach Injektion der Substanzkombination (Sic.Tox.3 + Sic.Enz.1).
Figur 5 zeigt die selektive Wirkung einer erfindungsgemäßen Zusammensetzung (Sic.Tox.2 + Sic. Enz.4) auf in einer Hautzellkultur befindliche Melanomzellen.
Figur 6 zeigt den gleichen Ausschnitt wie Figur 5, jedoch 3 Stunden nach Injektion.

### Beispiele

### Beispiel 1: Herstellung erfindungsgemäßer pharmazeutischer Zusammensetzungen

Zur manuellen Melkung wurden subadulte bzw. adulte Weibchen der Sicarius-Spinnenarten Sicarius oweni, Sicarius testaceus, Sicarius hahni oder Sicarius albospinosus mit den Fingern einer Hand in Rückenlage fixiert, dabei mit einer in der anderen Hand befindlichen sterilen Spritze (2 ml Brauninject v. Fa. B.Braun) mit aufgesetzter steriler Kanüle (20 oder 21 v. Becton Dickinson), die Tageszeit spielte keine Rolle, bei einer Raumtemperatur von 21 bis 27 Grad Celsius, bei einer Luftfeuchtigkeit von 50 bis 70 %, an den Chelizeren durch Berühren mit der stumpfen Seite der Kanüle zur Abgabe des Giftes stimuliert.

Dabei war es bevorzugt, daß die Stimulationszeit nicht länger als 90 Sekunden dauerte, da ansonsten das Tier einem unnötigen Stress ausgesetzt würde. Nach Erscheinen des Gifttropfens an den Giftklauen, wurde dieser mit der Spritze über die Kanüle aufgezogen. Für jedes Tier wurde eine neue Spritze mit neuer Kanüle verwendet. Anschließend wurde die Kanüle mit der Kanülenschutzhülle wieder verschlossen. Die verschlossene Spritze samt aufgezogenem Gift wurde direkt anschließend in einen Exsikator verbracht. Dieser wurde dann für mindestens 12 Stunden in einer Tiefkühltruhe bei mindestens minus 14 Grad Celsius aufbewahrt.

Der Spritze mit dem gefrorenen Gesamtgift wurde nach Entnahme aus der Tiefkühltruhe die Kanülenschutzhülle entfernt. Die Kanüle wurde in Lösungsmittel z.B. Proteinlösungsmittel von Fa. Carl Roth Gmbh & Co, KG (Lösungsmittel für Protein-Säulenchromatographie: 0,25 M Tris/HCl, pH 6,5 bis 7,3, 1,92 M Glycin, in destilliertem, deionisiertem Wasser/ wegen Denaturierung wird kein SDS im Puffer verwendet) eingetaucht und 1 mL aufgezogen. Dadurch wurde Gift in Lösung erhalten. Im Anschluß wurde die Kanüle entfernt. Die so aufbereiteten einzelnen Giftlösungen in Spritzen (5 Stück) wurden durch Ausdrücken (Ausspritzen) in einem sterilen, sauberen Teflonvial bei Raumtemperatur gesammelt. Das verschlossene Teflonvial wurde anschließend auf einem Vortex ohne Schaumbildung 30 Sekunden lang geschüttelt, wobei eine homogene Lösung erhalten wurde.

Nach der Durchmischung wurde die gesamte Lösung über einen Plexiglastrichter (um Kontamination zu vermeiden) in eine stehende transparente Plexiglassäule, die einen Innendurchmesser von 1,5 cm, eine Wandstärke von 2 mm und einer Höhe von 50 cm aufwies und unten konisch bis auf 1,5 mm zulaufend, offen war, gefüllt mit 20 mL Gel (Fa. Sigma/Supelco, AcA 34; Matrix: 3 % Acrylamid/ 4 % Agarose; Fraktionierungsbereich (MW): Proteine: 20 bis 350 kDa; Ausschlußgrenze: 750 kDa; Kügelchendurchmesser: 60 - 140 Micrometer), eingebracht. Die so eingebrachte Giftlösung durchlief das Gel und verdrängte dabei den im Gel befindlichen Puffer.

Nach vollständigem Eindringen der Giftlösung in das Gel, wurden zusätzliche 165 mL Lösungsmittel (0,25 M Tris/HCl, pH 6,5 bis 7,3, 1,92 M Glycin) auf die Säule gebracht. Dieses zusätzliche Lösungsmittel verdrängt bei ihrem Durchlauf durch das Gel die darin befindliche Giftlösung. Die ersten 15 mL, die unten aus der Säule liefen, waren Restpuffer und wurden verworfen. Nach diesen 15 mL wurden 40 Fraktionen zu je 4 mL aufgefangen. Die Trennung in jeweils 4 mL war bedingt durch die physikalischen und chemischen Eigenschaften der einzelnen Fraktionen, nachgewiesen durch Elektrophorese, bevorzugt SDS-PAGE. Als Auftragspuffer zum Peptidbindungs- und Proteinschutz wurden Roti Load 1+2 (Carl Roth GmbH & Co. KG, Karlsruhe: SDS-, Glycerol-, Bromphenolblau-, Phosphatpuffer, Roti Load 1 mit Mercaptoethanol, Roti Load 2 ohne Mercaptoethanol) verwendet. Die einzelnen Fraktionen wurden in steril sauberen verschraubbaren 5 mL Teflonvials getrennt aufgefangen. Die Qualitätskontrolle der Einzelfraktionen erfolgte mittels Elektrophorese.

Für die erfindungsgemäßen pharmazeutischen Zusammensetzungen wurden verschiedene Kombinationen der Fraktionen 1 bis 12 verwendet. Diese Fraktionen enthielten Spinnengift-Proteinbestandteile in einem Molekulargewichtsbereich von ca. 75 bis 175 kDa.

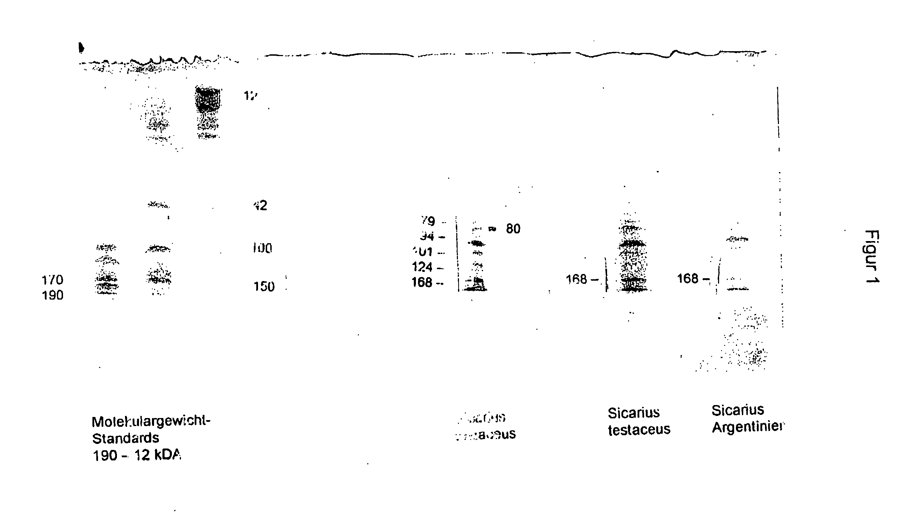

Eine SDS-PAGE-Elektrophorese des Gesamtgiftcocktails von Sicarius testaceus ist in Figur 1 gezeigt.

Um die Struktur der Substanzen aufzuklären, wurden die einzelnen Fraktionen über eine HPLC-MS-MS sowie über eine DAD-UV-Spektrometrie (DAD bzw. DADI: Direct Analysis of Daughter Ions, direkte Analyse von Tochterionen) untersucht. Bekannte Substanzen konnten in höherem Molekulargewichtsbereich (ab 10.000 Da) nicht dargestellt werden. Allerdings deuten die Grundgerüstbestimmungen auf die Zugehörigkeit der Substanzen zum Polypeptidtyp mit toxischen Komponenten (= Polypeptidtoxinen), und andererseits zum Phospholipase- und Hyaluronidase-Typ, hin.

Fraktionen mit gleicher Zusammensetzung können zusammen gesammelt werden. Für die weitere Verarbeitung und Lagerung wurden die einzelnen Fraktionen gefriergetrocknet, bspw mit folgenden Parametern:

Die zu lyophilisierende Fraktion wurde in einem offenen, mit perforierter Aluminiumfolie locker bedeckten Teflonvial auf minus 22 Grad Celsius gekühlt. Zur sicheren Durchfrierung der Probe wurde eine Kühlzeit von 11 Stunden eingehalten. Dann wurde ein Vakuum von 0,200 mbar angelegt. Nach Erreichen des Vakuums wurde die Fraktion auf plus 4 Grad Celsius erwärmt und mindestens 24 Stunden unter Aufrechterhaltung des Vakuums auf dieser Temperatur gehalten. Nach dem Gefriertrocknungsvorgang wurde das Teflonvial mit der lyophiliserten Fraktion luftdicht verschraubt. Die Lagerzeit beträgt bei Raumtemperatur ca. 3 Monate, bei plus 7 Grad Celsius ca. 1 Jahr und bei minus 14 Grad Celsius ca. 5 Jahre.

Substanzinhalt, -wirkung und Molekulargewicht der Fraktionen können der folgenden Tabelle entnommen werden.

**Tabelle 1**

| Fraktion-Nr. | mL von bis | Substanzbezeichnung | Substanzwirkung | | Molekulargewicht/kDa²⁾ |
|---|---|---|---|---|---|
| | | | Zellgift¹⁾ | Enzym¹⁾ | |
| 1 | 1-4 | Sic.Tox.1 | X | | ca. 72 |
| 2 | 5-8 | Sic.Tox.2 | X | | ca. 79-80 |
| 3 | 9-12 | Sic.Enz.1 | | X | |
| 4 | 13-16 | Sic.Tox.3 | X | | ca. 94 |
| 5 | 17-20 | Sic.Enz.2 | | X | |
| 6 | 21-24 | Sic.Enz.3 | | X | |
| 7 | 25-28 | Sic.Tox.4 | X | | ca. 101 |
| 8 | 29-32 | Sic.Enz.4 | | X | |
| 9 | 33-36 | Sic.Tox.5 | X | | ca. 124 |
| 10 | 37-40 | Sic.Tox.6 | X | | ca. 168 |
| 11 | 41-44 | Sic.Enz.5 | | X | |
| 12 | 45-48 | Sic.Enz.6 | | X | |

| | | | | | |
|---|---|---|---|---|---|
| ¹⁾ Substanzwirkung bestimmt über GC-MS-MS bzw. durch in vitro-Versuche mit verschiedenen Zellinien (vgl. Beispiel 2) | | | | | |
| ²⁾ Molekulargewicht bestimmt über HPLC-MS | | | | | |

Bei Anwendung der in diesem Beispiel angegebenen Reinigungsparameter waren dieses Elutionsprofil und die Zusammensetzungen der Fraktionen weitgehend reproduzierbar.

Die Fraktionen 13-40 besitzen ein Molekulargewicht bis ca. 350 KDa. Je höher molekular diese Giftbestandteile sind, desto schwieriger sind sie für weitere Untersuchungen in Lösung zu bringen. Erste Versuche haben jedoch gezeigt, dass die Gesamtrestfraktion (13 - 40) zum Beispiel menschliche Knochenhautzellen lysieren kann. Weiterhin kann bei der Austestung des Restgiftcocktails an verschiedenen humanen Zelllinien der Abbau von Kollagenen beobachtet werden. Ferner gelang über die Wirkung der Nachweis, dass darin Insektotoxine zu finden sind. Der lösliche Teil dieser Fraktionen wirkt auf Drosophila-, Schistocerca- und Locusta-Zelllinien zerstörend. Nach diesen Vorversuchen ist zwar derzeit nicht zu erwarten, daß ein oder mehrere der Fraktionen 13 bis 40 Substanzen aufweisen, die für die erfindungsgemäßen pharmazeutischen Zusammensetzungen verwendet werden können, dies ist aber erfindungsgemäß nicht ausgeschlossen.

### Beispiel 2: Tumorzell-zerstörende Wirkung der erfindungsgemäßen pharmazeutischen Zusammensetzungen

Derzeit ist es nicht möglich, auf Grund der vielfältigen Arten der Pathogenese von Tumorerkrankungen eine umfassende Präventivtherapie anzubieten. So steht bei lokal diagnostizierten Tumoren (primäre oder nachfolgende) die operative Entfernung an erster Stelle. Ein sich hierbei zeigendes Problem ist die Metastasierung, wenn nicht vollständig entferntes Tumorgewebe beim Durchschneiden in Kontakt mit Luft kommt. Dieses Problem kann durch das Aufbringen erfindungsgemäßer pharmazeutischer Zusammensetzungen auf die Schnittflächen bei einer operativen Tumorentfernung gelöst werden.

Für die im folgenden beschriebenen Versuchsreihen wurden die nachstehenden Tumorzelllinien verwendet:
Mamma-Ca- Mischzellenkultur: Dieser Zelltyp wurde aus einem Tumorzellengemisch von 11 Patientinnen herauskultiviert und seit 1989 in Langzeit- und Subkulturen beobachtet.
Lungen-Ca: 2 Zelllinien (1 männlicher und 1 weiblicher Patient) vom Labor KKH Weißenburg identifiziert und seit 1987 ebenfalls als Langzeit- und Subkultur beobachtet.
Malig. Melanom: Sicher als diese identifizierte Zellen vom Patienten nach Absprache mit diesem erhalten seit 1995 ebenfalls als Langzeit- und Subkultur beobachtet.
Prostata-Adenom: Mischzellkultur von 3 Patienten seit 1988 in Langzeit- und Subkultur beobachtet. In vitro bisher kein Therapieerfolg.
Gebärmutter Ca: Mischzellenkultur von 5 Patientinnen seit August 2000 in Kultur.
Eierstock-Ca: Mischzellenkultur von 3 Patientinnen seit August 2000 in Kultur.
Adeno-Ca: Mischzellenkultur von 3 Patienten seit August 2000 in Kultur.
Lebermetastasen: Mischzellenkultur von 2 Patienten ausgehend von kleinzelligem Bronchialcarcinom als Primärtumor; seit September 2000 in Kultur.
Lungenmetastasen: Mischzellenkultur von 2 Patientinnen ausgehend von Mamma-Ca als Primärtumor; seit August 2000 in Kultur.

### a) Wirkung des Gesamtgiftcocktails

Zu Beginn der Versuchsreihen wurde der gemolkene frische, bzw. gelöste Gesamtgiftcocktail und dessen Wirkung in Abhängigkeit von der Konzentration auf unterschiedliche Zelllinien ausgetestet. Dabei konnten keine befriedigenden Ergebnisse erreicht werden, da das Gift immer die gesamten Zellkulturen abtötete, wobei die für das Abtöten benötigte Zeit mit zunehmender Konzentration des eingesetzten Gifts abnahm.

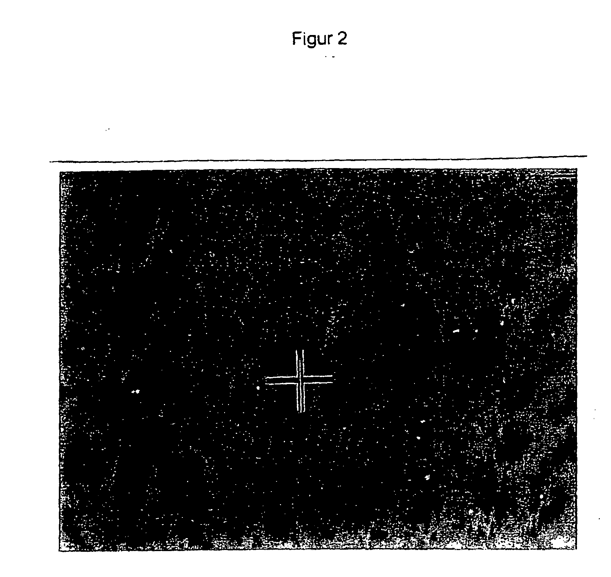

Die Wirkung des Sicarius-Gesamtgiftcocktails auf eine Mischkultur von gesunden Hautzellen mit Melanomzellen ist in Figur 2 beispielhaft dargestellt. Das Kulturmedium war DMEM/Ham's F-12. Man erkennt nur vereinzelt helle und dunkle Flecken, die Zellreste darstellen. Dies sind die einzigen Überbleibsel, die am Kulturflaschenboden, etwa 11 Stunden nach Injektion des Gesamtgiftcocktails vorgefunden werden.

### b) Wirkung verschiedener Einzel-Fraktionen des Sicarius-Spinnengiftcocktails

### Versuchsdurchführung:

Um die zellzerstörende/n Substanz bzw. Substanzen ausfindig zu machen, wurde der Gesamtgiftcocktail säulenchromatographisch, wie in Beispiel 1 beschrieben, fraktioniert und eine Molekulargewichtsbestimmung der einzelnen Komponenten über Elektrophorese durchgeführt. Die einzelnen Fraktionen (im Mittel 40) wurden an verwandten Zellkulturlinien, d.h. an gesunden Zellinien, im Vergleich zu den korrespondierenden tumorösen/entarteten Zellen unter gleichen Bedingungen vergleichsweise ausgetestet. Dabei wurden jeweils an bestimmten Bereichen Tumorzellen in Kulturen gesunder Zelllinien eingefügt. Anschließend wurden die Fraktionen, gelöst in isotonischer Kochsalzlösung, auf die Bereiche mit Tumorzellen und zum Vergleich auch auf Bereiche mit gesunden Zellen aufgetragen (injiziert) und die Wirkung auf die behandelten Zellen wurde lichtmikroskopisch verfolgt.

Hierbei konnte beobachtet werden, dass etwa 40 % der in den einzelnen Fraktionen befindlichen untersuchten Substanzen gewebezerstörende Wirkung aufweisen. Diese Substanzen können überdies in vitro konzentrationsabhängig bestimmte, mit Tumorzellen bedeckte Bereiche lysieren. Nach Ausschwemmung der Gifte mit dem normalen Mediumwechsel wuchsen diese Bereiche wieder zu. Lediglich bei 1 von 8 Kulturen (12,5%) trat anschließend wieder eine Zellentartung auf.

### c) Wirkung von Kombinationen verschiedener Fraktionen des Sicarius Spinnengiftcocktails

Wie oben bereits beschrieben, wurde festgestellt, daß es sich bei den antagonistisch wirkenden Substanzen im wesentlichen um die Enzyme von Hyaluronidasen- und Phospholipasen-Typ handelt.

Da diese Enzymtypen auch als Durchdringungsenzyme beschrieben sind und die Phospholipasen im speziellen auch als Immunhilfsstoffe gegen genetisch defekte Zellen im Organismus vorhanden sind, wurden nun Versuche mit Substanzkombinationen aus den Enzymen und Peptidtoxinen durchgeführt. Hierbei wurde wegen der besseren Handhabbarkeit zunächst mit den Fraktionen 1-12 experimentiert. Die Versuche erfolgten dabei wie unter Beispiel 2b) beschrieben, jedoch wurden keine Einzelfraktionen eingesetzt sondern Kombinationen von Fraktionen. Lösungsmittel war wiederum isotonische Kochsalzlösung.

### Pharmazeutische Zusammensetzung mit Substanzkombination 1:

Substanzkombination 1 enthielt 10 Gew.-% Sic. Enz. 2, 15 Gew.-% Sic.Tox.5 und 75 Gew.-% einer isotonischen Kochsalzlösung.

Die Wirkung von Substanzkombination 1 (vgl. auch Tabelle 4, V5) auf in Brustgewebezellen befindliche Mamma-Ca-Zellen war derart, daß nach Injektion der Substanzkombination im Bereich der Mamma-Ca-Zellen alle Brustkrebszellen zerstört wurden, während die gesunden Brustgewebezellen verschont blieben. Überdies wurde beobachtet, daß die durch die Substanzkombination 1 lysierte Fläche wieder zuwuchs.

Durch die molekulare Struktur und die physikalischen Eigenschaften der Substanzkombination 1 wird die Krebszelle von den Enzymen durch ihre natürlichen Eigenschaften aufgespürt und erkannt. Es kann vermutet werden, daß die Peptidtoxine über deren Eindringen in die Krebszelle die krebszelleigene Kommunikation (Transmitterzerstörung) vernichten. Auf Grund ihrer Molekularstruktur greifen die meisten der im Sicariustoxin vorhandenen Peptidtoxine Proteine bzw. proteinähnliche Verbindungen an. Krebszellen haben eine raffinierte Abwehr gegen das körpereigene Immunsystem ihres Wirtes/Trägers entwickelt, bei welcher Botenstoffe wie Interferone, Tumornekrosefaktoren und Zytokine eine wichtige Rolle spielen. Da diese Botenstoffe im flüssigen Medium vorhanden sind, bieten sie eine optimale Angriffsfläche für die ebenfalls im flüssigen Medium eingesetzten Peptidtoxine. Da nachweislich die von den erfindungsgemäßen Peptidtoxinen stark geschädigte Krebszellen nicht mehr teilungsfähig sind und benachbarte Krebszellen keine Schutzmechanismen gegen die Peptidtoxine entwickeln können, kann man eine Kommunikationszerstörung der Krebszellen untereinander vermuten.

Bei pharmazeutischen Zusammensetzungen, enthaltend Substanzkombination 1, sind die Enzyme in Bezug auf die Gesamtzusammensetzung quantitativ relativ stark angereichert.

### Pharmazeutische Zusammensetzung mit Substanzkombination 2:

Substanzkombination 2 enthielt 5 Gew.-% Sic.Enz. 1, 5 Gew.-% Sic.Tox. 3 und 90 Gew.-% isotonische NaCl-Lösung.

Bei Substanzkombination 2 kann eine allgemeine, räumlich optimal zu begrenzende Zellzerstörung erreicht werden. Hierbei können die gewählten Peptidtoxine das bearbeitete Gewebe zerstören. Die Beimischung der natürlichen peptidverdauenden Enzyme kontrolliert die räumlich und zeitlich zellzerstörende Wirkweise der Peptidtoxine.

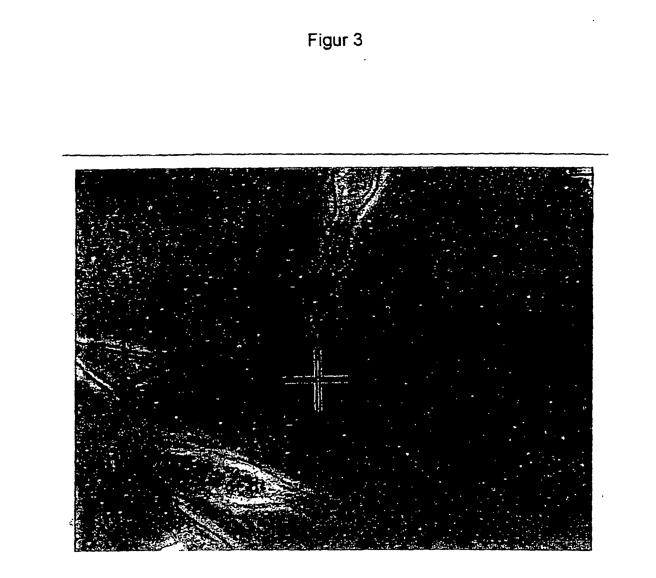

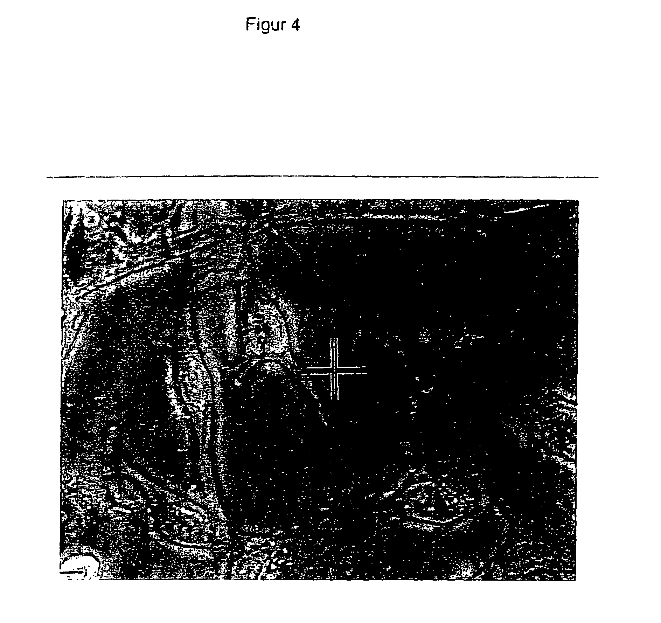

Die Wirkung von Substanzkombination 2 auf in einer Brustgewebezellkultur befindliche Mamma-Carcinom-Zellen ist in den Figuren 3 und 4 gezeigt. Auf der Aufnahme in Fig. 3 sind gesunde Brustgewebezellen erkennbar, die in DMEM/Ham's F-12 Medium angezogen wurden. In der linken Bildhälfte wuchsen ursprünglich Mamma-Carcinom-Zellen, die durch die Peptid-Enzym-Kombination zerstört wurden und nach 48 Stunden mit dem zweiten Mediumwechsel ausgespült wurden. Die gesunden Zellen wurden nicht beeinträchtigt und beginnen, in den entstandenen Freiraum hereinzuwachsen. Fig.4 zeigt den gleichen Ausschnitt wie Figur 3, jedoch 72 Stunden nach Injektion. Die linke, ehemals leere Fläche beginnt wieder mit gesunden Zellen zuzuwachsen (neben dem Kreuz). Zunächst entsteht erkennbar ein loser Zellverband, der dann durch echtes Gewebe wieder ergänzt wird.

Diese Beispiele zeigen, daß die Anwendung der Kombinationen 1 oder 2 abhängig von der Art, Größe und Lokalisation des zu entfernenden Tumors gewählt werden kann. Substanzkombinationen 1 und 2 können auf die Schnittflächen bei Tumoroperationen aufgebracht werden. Die Anwendung von Kombination 1 kann vorteilhaft bei Operationen sein, bei denen möglichst wenig gesundes angrenzendes Gewebe zerstört werden soll. Die Anwendung von Substanzkombination 2 kann bei Operationen vorteilhaft sein, bei denen auf das umliegende Gewebe nicht so viel Rücksicht genommen werden muss.

Der Operateur kann sich bei der Mischung der Substanzen in Bezug auf Qualität und Quantität, je nach Art und Lokalität des Tumors, der beispielhaft beigefügten Tabellen 2 und 3 bedienen, die auch eine weitere Substanzkombination 3 enthalten.

**Tabelle 2**

| Tumorart | Substanzmischung |
|---|---|
| Mamma-Ca | S 1 |
| Lungen-Ca | S 2 |
| Melanom, malig. | S 3 |
| S 1 = Sic.Enz.2 + Sic.Tox.5 | |
| S 2 = Sic.Enz.1 + Sic.Tox.3 | |
| S 3 = Sic.Enz.4 + Sic.Enz.5. + Sic.Tox.6 | |

**Tabelle 3**

| **Lösungsverhältnis in isotonischer Kochsalzlösung (MN)** | | | | | | | |
|---|---|---|---|---|---|---|---|
| Substanzmischung | Tumorgröße in mm | | | | | | |
| | 0-1 | 2-3 | 4-5 | 6-10 | Substanz % | Substanz % | Substanz % |
| S 1 | | | X | | 10 | 15 | ---- |
| S 2 | | X | | | 5 | 5 | ---- |
| S 3 | | | | X | 20 | 15 | 15 |

### Versuche zur Wirkungsweise weiterer Substanzkombinationen

In den nachfolgend angegebenen Tabellen 4 bis 12 sind die Wirkungen weiterer Substanzkombinationen auf Mamma-Ca-Zellen, Lungen-Ca-Zellen, Melanomzellen, Gebärmuter-Ca-Zellen, Eierstock-Ca-Zellen, Adeno-Ca-Zellen, Lebermetastasen, ausgehend von kleinzelligem Bronchialcarcinom als Primärtumor und Lungenmetastasen, ausgehend von Mamma-Ca als Primartumor, dargestellt. Die Versuche wurden wie oben beschrieben durchgeführt. Die eingesetzten Substanzmengen betrugen bei diesen Versuchen: 2 mg Peptidtoxin und 2 mg Enzym /100ml 0,9%-iger Kochsalzlösung.

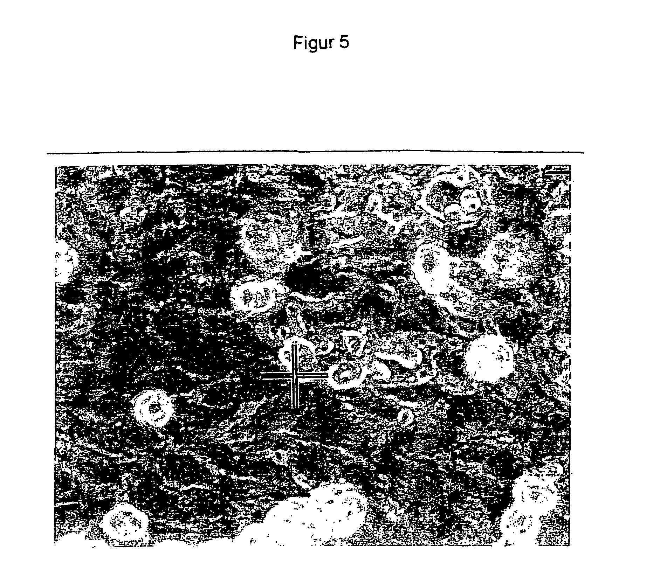

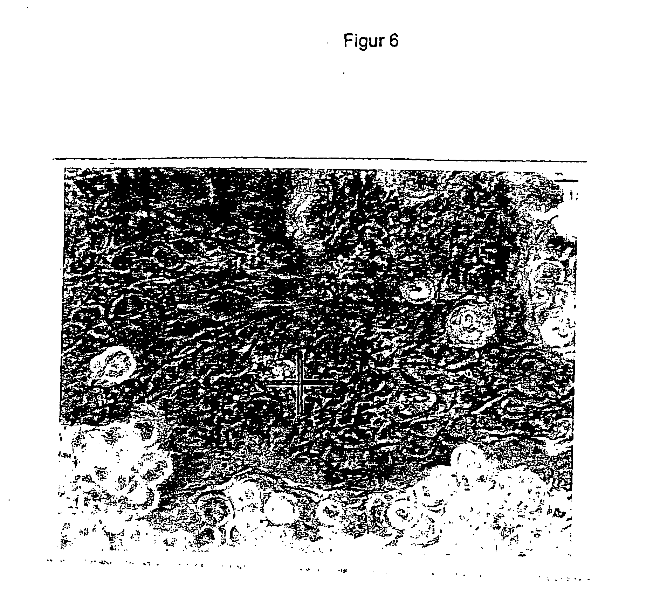

Beispielhaft ist in den Fig. 5 und 6 die Wirkung der Substanzkombination (Sic.Tox.2 + Sic.Enz.4) aus Tabelle 7 auf Melanomzellen gezeigt. Die Aufnahme zeigt einen Ausschnitt einer Hautzellkultur, herangezogen in DMEM/Ham's/F12, an der an verschiedenen Stellen Melanomzellen der gleichen Person geimpft wurden, 30 Minuten nach Injektion der Peptidtoxin-Enzymkombination. In der Mitte des Bildes sind noch unregelmäßige Melanomzellen sichtbar, welche alle nach Giftinjektion in Lyse begriffen sind. Vereinzelte runde, gesunde Hautzellen wurden vom Peptidtoxin-Enzymgemisch nicht angegriffen und sind deshalb noch sichtbar. Figur 6 zeigt den gleichen Ausschnitt wie Figur 5, jedoch 3 Stunden nach Injektion. Es ist gut erkennbar, daß alle Tumorzellen aufgelöst wurden. An der Grenze vom Tumorbereich zum gesunden Gewebe sind nur noch gesunde Hautzellen erkennbar, und im ehemaligen Tumorbereich sind einzelne gesunde Hautzellen erkennbar.

Es wurde weiterhin festgestellt, daß bei Langzeitkulturen (dickere Zellschicht in vitro) auch eine Zerstörung von Tumorgewebe in unteren Zellschichten erreicht wurde wenn die Enzyme in höherer Konzentration zugegeben wurden und diese das Gewebe für die Gifte augenscheinlich durchdringbarer und durch ihre Spezifität die genetisch defekten Zellen für das Gift angreifbar machten.

In den Tabellen 4 bis 12 sind die Ergebnisse der Einwirkung verschiedener Substanzkombinationen auf verschiedene Zellinien zusammenfassend dargestellt.

**Tab.4:**

| Mamma-Ca | | |
|---|---|---|
| Versuchs-Nr. Substanzkombination | | Ergebnis |
| V 1 | Sic.Tox.1+Sic.Enz.2 | Alle Brustkrebszellen abgetötet |
| V 2 | Sic.Tox.2+Sic.Enz.2 | " |
| V 3 | Sic.Tox.3+Sic.Enz.2 | Keine Wirkung an Zellen feststellbar |
| V 4 | Sic.Tox.4+Sic.Enz.2 | " |
| V 5 | Sic.Tox.5+Sic.Enz.2 | Alle Brustkrebszellen abgetötet Zerstörte lysierte Fläche wächst wieder zu |
| V 6 | Sic.Tox.6+Sic.Enz.2 | Keine Wirkung an Zellen feststellbar |

**Tab.5:**

| Mamma-Ca | | |
|---|---|---|
| Versuchs-Nr. Substanzkombination | | Ergebnis |
| V 1 | Sic.Tox.1+Sic.Enz.1 | Alle Zellen abgetötet |
| V 2 | Sic.Tox. 2+Sic.Enz.1 | Keine Wirkung an Zellen feststellbar |
| V 3 | Sic.Tox.3+Sic.Enz.1 | Alle Brustkrebszellen abgetötet. Keine Wirkung an Brustzellen feststellbar. |
| V 4 | Sic.Tox.4+Sic.Enz.1 | Keine Wirkung an Zellen feststellbar |
| V 5 | Sic.Tox.5+Sic.Enz.1 | Keine Wirkung an Zellen feststellbar |
| V 6 | Sic.Tox.6+Sic.Enz.1 | Alle Zellen abgetötet |
| V 7 | Sic.Tox.7+Sic.Enz.1 | Alle Zellen werden abgelöst und werden schwarz. |

**Tab.6:**

| Lungen-Ca | | |
|---|---|---|
| Versuchs-Nr. Substanzkombination | | Ergebnis |
| V 1 | Sic.Tox.1+Sic.Enz.6 | Alle Zellen in der Kultur werden abgetötet |
| V 2 | Sic. Tox.2+Sic.Enz.6 | Alle Lungenkrebszellen werden abgetötet. Zerstörte lysierte Fläche wächst sehr gut wieder zu. |
| V 3 | Sic.Tox.3+Sic.Enz.6 | " |
| V 4 | Sic.Tox.4+Sic.Enz.6 | Alle Lungenkrebszellen werden abgetötet. Die zerstörte lysierte Fläche wächst wieder zu. |
| V 6 | Sic.Tox.6+Sic.Enz.6 | Keine Wirkung an Zellen feststellbar |

**Tab.7:**

| Melanomzellen | | |
|---|---|---|
| Versuchs-Nr. Substanzkombination | | Ergebnis |
| V 1 | Sic.Tox.1+Sic.Enz.4 | Keine Wirkung an Zellen feststellbar |
| V 2 | Sic.Tox.2+Sic.Enz.4 | Alle Melanomzellen werden abgetötet. Die lysierte Fläche wird wieder von gesundem Hautgewebe bewachsen. |
| V 3 | Sic.Tox.3+Sic.Enz.4 | Alle Melanomzellen werden abgetötet. Die lysierte Fläche wächst nicht wieder zu. |
| V 4 | Sic.Tox.4+Sic.Enz.4 | Alle Hautzellen werden abgetötet. |
| V 5 | Sic.Tox.5+Sic.Enz.4 | " |
| V 6 | Sic.Tox.6+Sic.Enz.4 | Keine Wirkung an Zellen feststellbar |

**Tab.8:**

| Gebärmutter-Ca-Mischzellkultur von 5 Patientinnen | | |
|---|---|---|
| Versuchs-Nr. | Substanzkombination | Ergebnis |
| V1 | Sic.Tox.1 + Sic.Enz.2 | Alle entarteten Gebärmutterzellen |
| | | werden abgetötet. |
| V2 | Sic.Tox.2 + Sic.Enz.2 | Keine Wirkung an Zellen feststellbar |
| V3 | Sic.Tox.3 + Sic.Enz.2 | " |
| V4 | Sic.Tox.4 + Sic.Enz.2 | " |
| V5 | Sic.Tox.5 + Sic.Enz.2 | " |
| V6 | Sic.Tox.6 + Sic.Enz.2 | " |

**Tab.9:**

| Eierstock-Ca-Mischzellkultur von 3 Patientinnen | | |
|---|---|---|
| Versuchs-Nr. | Substanzkombination | Ergebnis |
| V1 | Sic.Tox.1 + Sic.Enz.2 | Alle Eierstock-Ca-Zellen werden |
| | | abgetötet. |
| V2 | Sic.Tox.2 + Sic.Enz.2 | Keine Wirkung an Zellen feststellbar. |
| V3 | Sic.Tox.3 + Sic.Enz.2 | " |
| V4 | Sic.Tox.4 + Sic.Enz.2 | " |
| V5 | Sic.Tox.5 + Sic.Enz.2 | " |
| V6 | Sic.Tox.6 + Sic.Enz.2 | " |

**Tab.10:**

| Adeno-Ca-Mischzellkultur von 3 Patienten | | |
|---|---|---|
| Versuchs-Nr. | Substanzkombination | Ergebnis |
| V1 | Sic.Tox.1 + Sic.Enz.6 | Keine Wirkung an Zellen feststellbar. |
| V2 | Sic.Tox.2 + Sic.Enz.6 | " |
| V3 | Sic.Tox.3 + Sic.Enz.6 | " |
| V4 | Sic.Tox.4 + Sic.Enz.6 | Alle Adeno-Ca-Zellen wurden lysiert. Die zerstörte, lysierte Zellfläche wächst langsam wieder mit gesunden Umgebungszellen zu. |
| V5 | Sic.Tox.5 + Sic.Enz.6 | Keine Wirkung an Zellen feststellbar. |
| V6 | Sic.Tox.6 + Sic.Enz.6 | ". |

**Tab.11:**

| Lebermetastasen, ausgehend von kleinzelligem Bronchialcarzinom als Primärtumor. Mischzellkultur von 2 Patienten | | |
|---|---|---|
| Versuchs-Nr. | Substanzkombination | Ergebnis |
| V1 | Sic.Tox.1+Sic.Enz.1+Sic.Enz.6 | Keine Wirkung an Zellen feststellbar |
| V2 | Sic.Tox.2+Sic.Enz.1+Sic.Enz.6 | Alle Lebermetastasen-Zellansammlungen wurden lysiert. Diese leeren Zellbereiche wachsen von aussen nach innen mit gesundem Umgebungsgewebe wieder zu. |
| V3 | Sic.Tox.3+Sic.Enz.1+Sic.Enz.6 | Ergebnis wie bei V2, mit deutlich verbesserter Regeneration des gesunden Gewebes. |
| V4 | Sic.Tox.4+Sic.Enz.1+Sic.Enz.6 | Keine Wirkung an Zellen feststellbar. |
| V5 | Sic.Tox.5+Sic.Enz.1+Sic.Enz.6 | " |
| V6 | Sic.Tox.6+Sic.Enz.1+Sic.Enz.6 | " |

**Tab.12:**

| Lungenmetastasen, ausgehend von Mamma-Ca als Primärtumor. Mischzellkultur von 2 Patientinnen | | |
|---|---|---|
| Versuchs-Nr. | Substanzkombination | Ergebnis |
| V1 | Sic.Tox.1+Sic.Enz.1+Sic.Enz.6 | Keine Wirkung an Zellen feststellbar. |
| V2 | Sic.Tox.2+Sic.Enz.1+Sic.Enz.6 | Die durch Lungenmetastasen ausgelösten Sekundärtumorzellansammlungen wurden vollständig lysiert. Nach 2-maligem Mediumwechsel wuchsen die leeren Zellbereiche wieder mit intakten Lungengewebszellen zu. |
| V3 | Sic.Tox.3+Sic.Enz.1+Sic.Enz.6 | Keine Wirkung an Zellen feststellbar. |
| V4 | Sic.Tox.4+Sic.Enz.1+Sic.Enz.6 | " |
| V5 | Sic.Tox.5+Sic.Enz.1+Sic.Enz.6 | " |
| V6 | Sic.Tox.6+Sic.Enz.1+Sic.Enz.6 | " |

### Beispiel 3: Enzym-Peptidtoxin-Blocktests

Weitere Versuche wurden dahingehend durchgeführt, um zu testen, ob bei vorgegebener Konzentration an Peptidtoxin und Enzym, bestimmte Enzymfraktionen bestimmte Peptidtoxin-Fraktionen neutralisierten.

### Versuchsdurchführung:

Bei den in den nachfolgenden Tabellen 13 und 14 zusammengefaßten Versuchen wurden Brustgewebszellen, Mamma-Ca Zellen, Hautzellen, Stammzellen, Leberzellen, Lungenzellen und Lungen-Ca Zellen sowie eine PHA-stimulierte Mischzellkultur aus verschiedenen Hautzelllininen verwendet.

Die untersuchten Zellen wurden mit 3 ml einer Lösung aus 2mg Enzym/100ml Medium (DMEM/Ham's F-12 mit ca. 1,2% Glutamin, 2,5% Penicillin/Streptomycin und 10% fetalem Kälberserum) versetzt. Nach 12 Stunden wurden 3ml einer Peptidtoxinlösung (2mg Peptidtoxin/100ml 0,9 %ige Kochsalzlösung) zugegeben. Die Auswirkungen auf die in der Zellkulturflasche befindlichen Zellen wurden lichtmikroskopisch beobachtet.
Wenn eine Neutralisation/Abblockung der Peptidtoxine erfolgte, wurde dies daran erkannt, daß das Peptidtoxin keine erkennbare Wirkung auf die in der Kultur befindlichen Zellen hatte, da es sofort durch das Enzym neutralisiert wurde. Andernfalls erfolgte eine Zerstörung/Lyse der Zellen.

Die in den nachfolgenden Tabellen zusammenfassend dargestellten Ergebnisse deuten darauf hin, dass bei den gewählten Konzentrationen und den oben angegebenen Zelllinien bestimmte Enzyme spezifisch immer die gleichen Peptidtoxine neutralisieren. Bei diesen Konzentrationsverhältnissen scheint eine Verwendung dieser Kombinationen bei den genannten Zellinien also vorteilhaft, da dadurch eine weite räumliche Ausdehnung der Peptidtoxine vermieden werden kann. Bei Änderung der Enzym- und Peptidkonzentration und bei Verwendung anderer Zelllinien können die Ergebnisse jedoch anders ausfallen.

### Tabellen 13 und 14: Enzym-Peptidtoxin-Blocktests

**Tabelle 13**

| Versuchs-Nr. Substanzkombination | | Ergebnis |
|---|---|---|
| V 1 | Sic.Tox.1+Sic.Enz.1 | |
| | Sic.Tox.2+Sic.Enz.1 | |
| | Sic.Tox.3+Sic.Enz.1 | Giftwirkung von Tox.3 wird mit Enz.1 neutralisiert |
| | Sic.Tox.4+Sic.Enz.1 | |
| | Sic.Tox.5+Sic.Enz.1 | |
| | Sic.Tox.6+Sic.Enz.1 | |
| | | |
| V 2 | Sic.Tox.1+Sic.Enz.2 | |
| | Sic.Tox.2+Sic.Enz.2 | |
| | Sic.Tox.3+Sic.Enz.2 | |
| | Sic.Tox.4+Sic.Enz.2 | |
| | Sic.Tox.5+Sic.Enz.2 | Giftwirkung von Tox.5 wird mit Enz.2 neutralisiert. |
| | Sic.Tox.6+Sic.Enz.2 | |
| | | |
| V 3 | Sic.Tox.1+Sic.Enz.3 | Giftwirkung von Tox.1 aber auch Tox.2 wird mit Enz.3 neutralisiert. |
| | Sic.Tox.2+Sic.Enz.3 | |
| | Sic.Tox.3+Sic.Enz.3 | |
| | Sic.Tox.4+Sic.Enz.3 | |
| | Sic.Tox.5+Sic.Enz.3 | |
| | Sic.Tox.6+Sic.Enz.3 | |

**Tabelle 14:**

| Versuchs-Nr. | Substanzkombination | Ergebnis |
|---|---|---|
| V 4 | Sic.Tox.1+Sic.Enz.4 | |
| | Sic.Tox.2+Sic.Enz.4 | Giftwirkung von Tox.2 aber auch von Tox.3 wird mit Enz.4 neutralisiert. |
| | Sic.Tox.3+Sic.Enz.4 | |
| | Sic.Tox.4+Sic.Enz.4 | |
| | Sic.Tox.5+Sic.Enz.4 | |
| | Sic.Tox.6+Sic.Enz.4 | |
| | | |
| V 5 | Sic.Tox.1+Sic.Enz.5 | Giftwirkung von Tox.6 aber auch |
| | Sic.Tox.2+Sic.Enz.5 | von Tox.1 und Tox.5 wird mit Enz.5 |
| | Sic.Tox.3+Sic.Enz.5 | neutralisiert. |
| | Sic.Tox.4+Sic.Enz.5 | |
| | Sic.Tox.5+Sic.Enz.5 | |
| | Sic.Tox.6+Sic.Enz.5 | |
| | | |
| V 6 | Sic.Tox.1+Sic.Enz.6 | |
| | Sic.Tox.2+Sic.Enz.6 | |
| | Sic.Tox.3+Sic.Enz.6 | |
| | Sic.Tox.4+Sic.Enz.6 | Giftwirkung von Tox.4 aber auch von Tox.2 und Tox.3 mit Enz.6 neutralisiert. |
| | Sic.Tox.5+Sic.Enz.6 | |
| | Sic.Tox.6+Sic.Enz.6 | |

### Beispiel 4: Kombination mit Schlangengift-Phospholipasen

In weitergehenden Versuchen konnte festgestellt werden, dass sich für einzelne Sicarius-Toxine auch spezielle Phospholipasen aus dem Gesamtgiftcocktail von Schlangen als Antagonisten verwenden lassen.

Es ist bekannt, dass auch im Gesamtgiftcocktail von Schlangen Enzyme vom Typ Hyaluronidase und Phospholipase (z.B. Phospholipase A1, Phospholipase D-B, Phospholipase E) vorkommen (Hankin E. H. (1899): The action of cobra poison on the blood (Path & Bact). Original in II. of Path. and Bact. 1897/Hankin E. H. (1900): The haemolytic action of snake toxins and toxic p. sera, 450. Med. Gazette/ Dennys G. W. P. (1916): Experimentes with snake venoms and some important notes. Med. Gazette/Ardeshire K. (1918): Bite from Echis carinata-recovery (corr.). Med. Gazette/ Neuberg Rosenberg (1907): Lipolyse, Agglutination und Hämolyse. B. kl. W. / Lamb and Hunler (1904-1907): Action of venoms of different species of poisonous snakes and the nervous system. Lancet/ Lindemann W. (1898): Über die Sekretionserscheinungen der Giftdrüse der Kreuzotter. Arch. f. mikr. Anat.) und eigene Versuche (Burda R., Richter R., Schrottenloher E., Weickmann D. (2001): Visueller Schnellnachweis für Enzyme in Schlangengiften unter Zuhilfenahme eines Schwarzweißfilmes, in press).

Da Schlangengifte in größerer Menge zu gewinnen sind und die darin enthaltenen Enzyme prozentual stärker vertreten sind, wurde die antagonistische Wirkung einzelner Enzymfraktionen aus Schlangengiften an den Sicariustoxinen ausgetestet. Hierbei wurden folgende Ergebnisse erhalten.

**Tab.15 :**

| Enzym-Peptidtoxin-Blocktests | | |
|---|---|---|
| Versuchs-Nr. | Substanzkombination | Ergebnis |
| V1 | Sic.Tox.1 + AtractaspisEnz.1 | Giftwirkung von Sic.Tox.1 wird mit Atractaspis Enz.1 neutralisiert. |
| | Sic.Tox.2 + AtractaspisEnz.1 | |
| | Sic.Tox.3 + AtractaspisEnz.1 | |
| | Sic.Tox.4 + AtractaspisEnz.1 | |
| | Sic.Tox.5 + AtractaspisEnz.1 | |
| | Sic.Tox.6 + AtractaspisEnz.1 | |
| | | |
| V2 | Sic.Tox.1 + Bit.Phos.A2 | |
| | Sic.Tox.2 + Bit.Phos.A2 | |
| | Sic.Tox.3 + Bit.Phos.A2 | Giftwirkung von Sic.Tox.3 wird mit Bit.Phos.A2 neutralisiert. |
| | Sic.Tox.4 + Bit.Phos.A2 | |
| | Sic.Tox.5 + Bit.Phos.A2 | |
| | Sic.Tox.6 + Bit.Phos.A2 | |
| | | |
| V3 | Sic.Tox.1 + Bit.Phos.D | |
| | Sic.Tox.2 + Bit.Phos.D | |
| | Sic.Tox.3 + Bit.Phos.D | |
| | Sic.Tox.4 + Bit.Phos.D | |
| | Sic.Tox.5 + Bit.Phos.D | |
| | Sic.Tox.6 + Bit.Phos.D | Giftwirkung von Sic.Tox.6 wird mit Bit.Phos.D neutralisiert. |
| | | |
| V4 | Sic.Tox.1 + Vip.asp.Phos.A1 | |
| | Sic.Tox.2 + Vip.asp.Phos.A1 | |
| | Sic.Tox.3 + Vip.asp.Phos.A1 | |
| | Sic.Tox.4 + Vip.asp.Phos.A1 | Giftwirkung von Sic.Tox.4 wird mit Vip.asp.Phos.A1 neutralisiert. |
| | Sic.Tox.5 + Vip.asp.Phos.A1 | |
| | Sic.Tox.6 + Vip.asp.Phos.A1 | |

**Sic.Tox.** ist jeweils wie oben definiert.

**AtractaspisEnz.1** steht für eine Phospholipase vom A-Typ aus dem Gift der Atractaspis bibronii.

**Bit.Phos.A2** steht für die Phospholipase A2 aus den Giften der dunklen südafrikanischen Populationen von Bitis arietans (evtl. andere Art, wie früher vorgeschlagen Bitis lachesis)

**Bit.Phos.D** steht für die Phospholipase D aus den Giften der dunklen südafrikanischen Populationen von Bitis arietans (evtl. andere Art, wie früher vorgeschlagen Bitis lachesis)

**Vip.asp.Phos.A1** steht für die Phospholipase aus den Giften der Vipera aspis zinnikeri

### Beispiel 5: Versuche mit Fraktionen weiterer Spinnenarten der Sicariidae

### Sicarius oweni

Hier wurde unter anderem ein Toxin der Sicarius oweni mit ca. 69 kDa, welches ähnliche Mamma-Ca zellzerstörende Eigenschaften wie das Sic.Tox.1 mit ca. 72 kDa und das Sic.Tox.5 mit ca. 124 kDa von Sicarius testaceus, aufweist, getestet. Als Phospholipase zur Kombination eignet sich die Enzymfraktion, die bei Sicarius oweni nach ca. 21-24 ml durch die Säule läuft. Die Bezeichnung der Fraktionen erfolgte wie bei Sicarius testaceus.

**Tab.16 :**

| **Mamma-Ca mit Fraktionen aus dem Gift der Sicarius oweni** | | |
|---|---|---|
| Versuchs-Nr. | Substanzkombination | Ergebnis |
| V1 | Sic.Tox.1 + Sic.Enz.1 | Keine Wirkung an Zellen feststellbar |
| V2 | Sic.Tox.2 + Sic.Enz.1 | Alle Brustkrebszellen werden abgetötet |
| V3 | Sic.Tox.3 + Sic.Enz.1 | Keine Wirkung an Zellen feststellbar |
| V4 | Sic.Tox.4 + Sic.Enz.1 | " |
| V5 | Sic.Tox.5 + Sic.Enz.1 | " |

### Loxosceles laeta

Hier wurde speziell für großzellige Lungen-Ca Zellen ein Peptidtoxin aus dem Gesamtgiftcocktail mit ca. 95 kDa und als Phospholipase die Enzymfraktion, die bei Loxosceles laeta nach ca. 37-40 ml durch die Säule läuft, eingesetzt.

**Tab.17 :**

| **Großzelliges Lungen Ca mit Fraktionen aus dem Gift der Loxosceles laeta** | | |
|---|---|---|
| Versuchs-Nr. | Substanzkombination | Ergebnis |
| V1 | Lox.Tox.1 + Lox.Enz.2 | Keine Wirkung an Zellen feststellbar |
| V2 | Lox.Tox.2 + Lox.Enz.2 | " |
| V3 | Lox.Tox.3 + Lox.Enz.2 | " |
| V4 | Lox.Tox.4 + Lox.Enz.2 | " |
| V5 | Lox.Tox.5 + Lox.Enz.2 | " |
| V6 | Lox.Tox.6 + Lox.Enz.2 | " |
| V7 | Lox.Tox.7 + Lox.Enz.2 | Alle Lungen-Ca Zellen wurden zerstört Die gesunden Lungengewebszellen lassen sich weiter kultivieren. |
| V8 | Lox.Tox.8 + Lox.Enz.2 | Keine Wirkung an Zellen feststellbar |

### Beispiel 6: Synergistische und zeitversetzt antagonistische Wirkung von Sic.Enz.2 auf Sic.Tox.5

Sic.Enz. 2 ist wie oben beschrieben selektiv für Mamma-Ca-Zellen (s. Substanzkombination1 und Tab. 4). Andererseits wurde Sic.Enz.2 auch als Sic.Tox.5 blockierend beschrieben (s. Tab. 13). Tabelle 4 und Tabelle 13 liegen aber zwei völlig verschiedene Versuche zu Grunde:

Tabelle 4 beschreibt die Kombination bezüglich der selektiven Abtötung von Tumorzellen in vitro. Hier verhält es sich so, dass das Sic.Enz.2 die entarteten Brustkrebszellen findetl, die Zellwand auflockert und für das Peptidtoxin zugänglich macht, so dass Sic.Tox.5 die Zelle infiltrieren und zerstören kann. Hier sind die beiden Substanzen erst kurz vor Zugabe in die Zellkultur zusammengemischt.

Tabelle 13 beschreibt zwar die gleiche Kombination der verwendeten Substanzen, jedoch im zeitlichen Abstand von 12 Stunden gemischt (zunächst Sic.Enz. 2, dann Sic.Tox.5).

Die Ergebnisse dieser Versuche widersprechen sich nicht, da man die Kombination nach Versuch 5, Tabelle 4 nicht mit dem hier beschriebenen zeitlich versetzten Enzym-Peptid-Blocktest nach Tabelle 8 vergleichen kann. Offenbar ist unter den Bedingungen des Enzym-Peptid-Blocktests Sic.Enz. 2 auch antagonistisch gegen Sic.Tox.5 wirksam.

### Beispiel 7: Weitere Versuche zur antagonistischen Wirkung

Generell ist zu sagen, dass es sich bei den Hyaluronidasen und Phospholipasen jeweils um eine Enzymgruppe/familie handelt (Cantore G., Bettini S. (1958): Contributo allo studio dell azione farmocologica dell veneno di L. tredecimguttatus Rossi. II Azione sulla musculatura bronchiale. Riv. Parasit. 19:297), innerhalb derer die einzelnen Mitglieder unterschiedliche Wirkweisen in Bezug auf die Qualität der abzubauenden Substanzen aufweisen.

Bislang sind diese Hyaluronidasen und Phospholipasen als Durchdringungsenzyme beschrieben (Heitz J.,Norment B. (1974): Characteristics of an alcaline phosphatase activity in brown recluse venom. Toxicon 12:181/Bernheimer A., et al (1985): Comparative toxinology of Loxosceles reclusa and Corynebacterium pseudotuberculosis. Science 228:590-591).

Im Rahmen dieser Erfindung stellte sich heraus, dass einzelne verwendeten Mitglieder dieser Enzymgruppen antagonistisch auf die eingesetzten Peptidtoxine wirken, wobei die zeitliche und räumliche Ausbreitung der Peptidtoxine begrenzt wird.

Der Nachweis über die antagonistische Wirkung wurde über die Bestimmung des Molekulargewichtes der verwendeten Substanzen im Vorher-Nachher-Vergleich mittels der von uns bereits beschriebenen Elektrophorese durchgeführt. Bei antagonistischer Wirkung fehlt die vorher vorhanden Bande für das Peptidtoxin.

In einem Versuch mit einer Mischzellkultur aus Mamma-Ca und gesunden Brustgewebszellen konnte nach gezielter Zugabe von Sic.Tox.1 mittels Feinspritze in markierte Mamma-Ca-zellreiche Gebiete folgendes festgestellt werden:

Sic.Tox.1 breitet sich ringförmig um den Einspritzbereich ca. 10 Stunden lang relativ gleichmäßig aus. Dabei werden sowohl die gesunden als auch die Tumorzellen lysiert. Dieser Vorgang ist leicht über ein Invertmikroskop zu beobachten. Nach diesen 10 Stunden tritt eine deutliche Verlangsamung der Ausbreitungsgeschwindigkeit des Sic.Tox.1 ein, wobei die spezifische zellzerstörende Wirkung von Six.Tox.1 weiterhin in Abständen von 1, 2 und 7 Tagen unter dem Invertmikroskop zu beobachten war, da sich der Bereich der Zellzerstörung zu diesem Zeitpunkt noch vergrößerte.

Wie in Tabellen 13 und 14 bereits dargestellt, wurde Sic.Tox.1 mit 6 verschiedenen Sic.Enzymen kombiniert. Dabei konnte festgestellt werden, dass bei Zugabe von Sic.Enz.3 die zelllysierende Wirkung von Sic.Tox.1 nach 9 Stunden vollständig gestoppt wurde. Die Elektrophorese des Mediums und eines Zelllysates ergab keine Bande bei 72 kDa (Molekulargewicht von Sic.Tox.1).

Über einen modifizierten Ouchterlony-Test konnte zudem eine "Antigen/Antikörper-Wirkung" von Sic.Enz.3 zu Sic.Tox.1 dargestellt werden, d.h. eine Bindung von Sic.Enz.3 an Sic. Tox.1. Zusätzlich konnte beobachtet werden, dass nach Neutralisation von Sic.Tox.1 durch Sic.Enz.3 die lysierten Bereiche wieder zuwuchsen.

## Patentansprüche

1. Pharmazeutische Zusammensetzung zur Behandlung von Tumorerkrankungen, enthaltend in einer pharmazeutisch wirksamen Menge:
c) zumindest ein Peptidtoxin, sowie
d) zumindest eine hierzu antagonistisch wirkende Substanz und/oder zumindest eine Durchdringungssubstanz,
wobei zumindest das Peptidtoxin aus dem Gift von Spinnen der Familie der Sicariidae stammt und wahlweise die hierzu antagonistisch wirkende Substanz und/oder die Durchdringungssubstanz aus dem Gift von Spinnen der Familie der Sicariidae stammt.

2. Pharmazeutische Zusammensetzung nach Anspruch 1,
**dadurch gekennzeichnet, daß**
ein oder mehrere weitere Substanzen aus dem Gift von Spinnen der Familie der Sicariidae enthalten sind.

3. Pharmazeutische Zusammensetzung nach Anspruch 1 oder 2,
**dadurch gekennzeichnet, daß**
das Peptidtoxin und die antagonistisch wirkende Substanz und/oder die Durchdringungssubstanz aus dem Gift von Sicarius-, Loxosceles-, Scytodes- und/oder Drymusa-Spinnenarten stammen.

4. Pharmazeutische Zusammensetzung nach einem oder mehreren der vorhergehenden Ansprüche,
**dadurch gekennzeichnet, daß**
die Sicarius-Spinnenarten Sicarius oweni, Sicarius testaceus, Sicarius hahni und Sicarius albospinosus, die Loxosceles-Spinnenarten Loxosceles rufescens, Loxosceles reclusa und Loxosceles laeta, und/oder die Scytodes-Spinnenarten Scytodes thoracica, Scytodes rufa und Scytodes longipes sind.

5. Pharmazeutische Zusammensetzung nach einem oder mehreren der vorhergehenden Ansprüche,
**dadurch gekennzeichnet, daß**
die antagonistisch wirkende Substanz und/oder das Durchdringungsenzym aus einem anderen Organismus als Spinnen der Familie der Sicariidae stammt.

6. Pharmazeutische Zusammensetzung nach einem oder mehreren der vorhergehenden Ansprüche,
**dadurch gekennzeichnet, daß**
als Peptidtoxin weiterhin zumindest ein Schlangengift, bevorzugt das Grubenottern-Schlangengift Captopril, enthalten ist.

7. Pharmazeutische Zusammensetzung nach einem oder mehreren der vorhergehenden Ansprüche,
**dadurch gekennzeichnet, daß**
als antagonistisch wirkende Substanz und/oder Durchdringungssubstanz zumindest eine Hyaluronidase aus Schlangengiften, bevorzugt aus Cobragiften, oder zumindest eine Phospholipase aus Actrataspis bibronii, Bitis arietans oder Vipera aspis zinnikeri enthalten ist.

8. Pharmazeutische Zusammensetzung nach einem oder mehreren der vorhergehenden Ansprüche,
**dadurch gekennzeichnet, daß**
die antagonistisch wirkende Substanz und/oder die Durchdringungssubstanz eine Phospholipase oder eine Hyaluronidase oder eine Kombination beider Substanzen ist.

9. Pharmazeutische Zusammensetzung nach einem oder mehreren der vorhergehenden Ansprüche,
**dadurch gekennzeichnet, daß**
die Durchdringungssubstanz eine Phospholipase ist.

10. Pharmazeutische Zusammensetzung nach einem oder mehreren der vorhergehenden Ansprüche,
**dadurch gekennzeichnet, daß**
das Peptidtoxin und die hierzu antagonistisch wirksame Substanz und/oder die Durchdringungssubstanz durch ein Fraktionierungsverfahren aus dem Spinnengift erhalten wurden.

11. Pharmazeutische Zusammensetzung nach einem oder mehreren der vorhergehenden Ansprüche,
**dadurch gekennzeichnet, daß**
das Peptidtoxin und die hierzu antagonistisch wirkende Substanz und/oder die Durchdringungssubstanz durch Gelchromatographie, HPLC, Affinitätschromatographie und/oder Ionenaustauschchromatographie aus dem Spinnengift erhalten wurden.

12. Pharmazeutische Zusammensetzung nach einem oder mehreren der vorhergehenden Ansprüche,
**dadurch gekennzeichnet, daß**
das Peptidtoxin und die antagonistisch wirkende Substanz und/oder die Durchdringungssubstanz wie folgt erhalten wurden:
bei Verwendung einer Säule mit einem Innendurchmesser von 1,5 cm und einer Höhe von 50 cm, die unten konisch bis auf 1,5 mm zuläuft und die mit 20 ml eines Gelchromatographiegels AcA 34, Matrix: 3% Acrylamid/4% Agarose, Fraktionierbereich: 20 bis 350 kDa, Ausschlußgrenze: 750kDa, Kügelchendurchmesser 60-140µm gefüllt ist, Spinnengift aus Sicarius oweni, Sicarius testaceus, Sicarius hahni oder Sicarius albospinosus in 0,25M Tris/HCl, pH 6,5 bis 7,3, und 1,92M Glycin in destilliertem, deionisiertem Wasser im homogenen Zustand auf das Gel aufgebracht wurde, wenn die Giftlösung das Gel durchlaufen hat und 165 ml einer Lösung von 0,25 M Tris/HCl, pH 6,5 bis 7,3, und 1,92M Glycin in destilliertem, deionisiertem Wasser aufgebracht wurden und die ersten 15 ml des Durchlaufs verworfen wurden und je 4 ml Fraktionen gesammelt werden, sich die Peptidtoxine in den Fraktionen 1, 2, 4, 7, 9 und 10 und sich die antagonistisch wirkenden Substanzen und/oder die Durchdringungssubstanzen in den Fraktionen 3, 5, 6, 8, 11 und 12 befinden.

13. Pharmazeutische Zusammensetzung nach einem oder mehreren der vorhergehenden Ansprüche,
**dadurch gekennzeichnet, daß**
sie ein Peptidtoxin und eine hierzu antagonistisch wirkende Substanz und/oder eine Durchdringungssubstanz enthält, die aus verschiedenen Fraktionen des Spinnengifts stammen.

14. Pharmazeutische Zusammensetzung nach einem oder mehreren der vorhergehenden Ansprüche,
**dadurch gekennzeichnet, daß**
das Peptidtoxin und die hierzu antagonistisch wirkende Substanz und/oder die Durchdringungssubstanz in einer solchen Menge vorliegen, daß eine bezüglich Tumorzellen zerstörende Wirkung des Peptidtoxins und der hierzu antagonistisch wirkenden Substanz und/oder der Durchdringungssubstanz vorliegt.

15. Pharmazeutische Zusammensetzung nach einem oder mehreren der vorhergehenden Ansprüche,
**dadurch gekennzeichnet, daß**
die Menge so ausgewählt ist, daß eine räumlich und/oder zeitlich kontrollierte Ausbreitung im Gewebe sichergestellt ist.

16. Pharmazeutische Zusammensetzung nach einem oder mehreren der vorhergehenden Ansprüche,
**dadurch gekennzeichnet, daß**
die Menge an Peptidtoxin und antagonistisch wirkender Substanz und/oder Durchdringungssubstanz in Abhängigkeit vom zu behandelnden Tumor gewählt ist.

17. Pharmazeutische Zusammensetzung nach einem oder mehreren der vorhergehenden Ansprüche,
**dadurch gekennzeichnet, daß**
sie übliche Träger- und Hilfsstoffe und/oder weitere Wirkstoffe enthält.

18. Pharmazeutische Zusammensetzung nach einem oder mehreren der vorhergehenden Ansprüche,
**dadurch gekennzeichnet, daß**
sie als übliche Träger- und Hilfsstoffe isotone Lösungen, Eiweißlösungen, Aminosäurelösungen und/oder keimtötende Lösungen, bevorzugt Ringerlösung, 0,9%-ige NaCl-Lösung, Human-Albuminlösung und/oder Glutaminlösung, enthält, und daß sie als weitere Wirkstoffe Antibiotika, Antimykotika, Antituberkulotika, Mittel gegen Parasiten, Aminosäuren, die Wundheilung begünstigende Enzyme, Mitosehemmstoffe und/oder Zytostatika enthält.

19. Pharmazeutische Zusammensetzung nach einem oder mehreren der vorhergehenden Ansprüche,
**dadurch gekennzeichnet, daß**
ein Derivat des Peptidtoxins und/oder der antagonistisch wirkenden Substanz und/oder der Durchdringungssubstanz in der pharmazeutischen Zubereitung enthalten ist und/oder das Peptidtoxin und/oder die hierzu antagonistisch wirkende Substanz und/oder das Durchdringungssubstanz chemisch-synthetisch oder durch rekombinante biotechnologische Methoden hergestellt ist und sie in ihrer Wirkung dem im Gift von Spinnen der Familie Sicariidae enthaltenen Toxinen oder den hierzu antagonistisch wirkenden Substanzen und/oder Durchdringungssubstanzen und Derivaten hiervon entsprechen.

20. Verfahren zur Herstellung einer pharmazeutischen Zusammensetzung, enthaltend in einer pharmazeutisch wirksamen Menge zumindest ein Peptidtoxin sowie zumindest eine hierzu antagonistisch wirkende Substanz und/oder zumindest eine Durchdringungssubstanz, wobei das Peptidtoxin aus dem Gift von Spinnen der Familie der Sicariidae stammt und wahlweise die hierzu antagonistisch wirkende Substanz und/oder die Durchdringungssubstanz aus dem Gift von Spinnen der Familie der Sicariidae stammt, mit den folgenden Schritten:
- Gewinnen eines Spinnengift-Rohgemischs durch an sich bekannte Verfahren sowie Fraktionierung der Mischung, um das Peptidtoxin und wahlweise die hierzu antagonistisch wirkende Substanz und/oder die Durchdringungssubstanz und wahlweise weitere Substanzen in möglichst voneinander getrennten Fraktionen zu erhalten;
- Kombination verschiedener Fraktionen des Peptidtoxins mit Fraktionen, die hierzu antagonistisch wirkende Substanzen und/oder Durchdringungssubstanzen enthalten, oder mit aus anderen Organismen stammenden antagonistisch wirkenden Substanzen und/oder Durchdringungssubstanzen, um eine pharmazeutisch wirksame Zusammensetzung zu erhalten.

21. Verfahren nach Anspruch 20,
**dadurch gekennzeichnet, daß**
das Spinnengift-Rohgemisch aus weiblichen Spinnen der Familie der Sicariidae gewonnen wird.

22. Verfahren nach den Ansprüchen 20 und 21,
**dadurch gekennzeichnet, daß**
das Spinnengift-Rohgemisch durch manuelles Melken erhalten wird.

23. Verfahren nach einem oder mehreren der Ansprüche 20 bis 22,
**dadurch gekennzeichnet, daß**
das Spinnengift-Rohgemisch vor der Fraktionierung homogenisiert wird.

24. Verfahren nach einem oder mehreren der Ansprüche 20 bis 23,
**dadurch gekennzeichnet, daß**
die Fraktionen vor der Weiterverarbeitung zu einer pharmazeutischen Zusammensetzung tiefgekühlt und weiter bevorzugt lyophilisiert werden.

25. Verwendung von pharmazeutischen Zusammensetzungen nach einem oder mehreren der Ansprüche 1 bis 19 in der Medizin.

26. Verwendung von pharmazeutischen Zusammensetzungen nach einem oder mehreren der Ansprüche 1 bis 19 zur Behandlung von Tumorerkrankungen und/oder zur unterstützenden Behandlung bei Tumoroperationen.

## Claims

1. A pharmaceutical composition for the treatment of tumor diseases containing in a pharmaceutically effective amount:
a) at least one peptide toxin, as well as
b) at least one substance having an antagonistic effect thereon and/or at least a penetrant,
wherein at least the peptide toxin is derived from the poison of spiders of the family of Sicariidae and optionally the substance having an antagonistic effect thereon and/or the penetrant is derived from the poison of spiders of the family of Sicariidae.

2. A pharmaceutical composition according to claim 1 **characterized in that** it contains one or more other substances from the poison of spiders of the family of Sicariidae.

3. A pharmaceutical composition according to claim 1 or 2 **characterized in that** said peptide toxin and said substance having an antagonistic effect and/or the penetrant are derived from the poison of Sicarius, Loxosceles, Scytodes, and/or Drymusa spider species.

4. A pharmaceutical composition according to one or more of the preceding claims, **characterized in that** the Sicarius spider species are Sicarius oweni, Sicarius testaceus, Sicarius hahni, and Sicarius albospinosus, the Loxosceles spider species are Loxosceles rufescens, Loxosceles reclusa, and Loxosceles laeta, and/or the Scytodes spider species are Scytodes thoracica, Scytodes rufa, and Scytodes longipes.

5. A pharmaceutical composition according to one or more of the preceding claims, **characterized in that** the substance having an antagonistic effect and/or the penetration enzyme is derived from an organism different from spiders of the family of Sicariidae.

6. A pharmaceutical composition according to one or more of the preceding claims, **characterized in that** at least one snake poison, preferably the pit viper poison captopril is contained as said peptide toxin.

7. A pharmaceutical composition according to one or more of the preceding claims, **characterized in that** as said substance having an antagonistic effect and/or said penetrant at least one hyaluronidase from snake poisons, preferably from cobra poisons, or at least one phospholipase from Actrataspis bibronii, Bitis arietans or Vipera aspis zinnikeri is contained.

8. A pharmaceutical composition according to one or more of the preceding claims, **characterized in that** said substance having an antagonistic effect and/or said penetrant is a phospholipase or a hyaluronidase or a combination of both substances.

9. A pharmaceutical composition according to one or more of the preceding claims, **characterized in that** said penetrant is a phospholipase.

10. A pharmaceutical composition according to one or more of the preceding claims, **characterized in that** said peptide toxin and said substance having an antagonistic effect thereon and/or said penetrant are obtained from the spider poison by a fractionation procedure.

11. A pharmaceutical composition according to one or more of the preceding claims, **characterized in that** said peptide toxin and said substance having an antagonistic effect thereon and/or said penetrant are obtained from the spider poison by means of gel chromatography, HPLC, affinity chromatography and/or ion exchange chromatography.

12. A pharmaceutical composition according to one or more of the preceding claims, **characterized in that** said peptide toxin and said substance having an antagonistic effect thereon and/or said penetrant have been obtained as follows:
Using a column with an inner diameter of 1.5 cm and a height of 50 cm tapering at the bottom to 1.5 mm, and which was filled with 20 ml of a gel chromatography gel AcA 34, matrix: 3% acrylamide/4% agarose, fractionation range: 20 to 350 Da, cut off limit: 750 kDa, bead diameter: 60-140 µm;
spider poison from Sicarius oweni, Sicarius testaceus, Sicarius hahni, or Sicarius albospinosus in 0.25 M Tris/HCl, pH 6.5 to 7.3, and 1.92 M glycine in destilled, deionized water was loaded onto the gel in a homogeneous form,
and after the poison solution passed through the gel and 165 ml of a solution of 0.25 M Tris/HCl, pH 6.5 to 7.3, and 1.92 M glycine in destilled, deionized water were loaded onto the column and the first 15 ml of the eluent were discarded and fractions of 4 ml each were collected, the peptide toxins are found in fractions 1, 2, 4, 7, 9, and 10 and the substances having an antagonistic effect thereon and/or the penetrants are found in fractions 3, 5, 6, 8, 11, and 12.

13. A pharmaceutical composition according to one or more of the preceding claims, **characterized in that** it contains a peptide toxin and a substance having an antagonistic effect thereon and/or a penetrant which are derived from different fractions of the spider poison.

14. A pharmaceutical composition according to one or more of the preceding claims, **characterized in that** said peptide toxin and said substance having an antagonistic effect thereon and/or the penetrant are present in a sufficient amount that a destructive effect with respect to tumor cells of the peptide toxin and the substance having an antagonistic effect thereon and/or the penetrant are obtained.

15. A pharmaceutical composition according to one or more of the preceding claims, **characterized in that** the amount is selected to ensure a spatially and/or temporally controlled distribution within the tissue.

16. A pharmaceutical composition according to one or more of the preceding claims, **characterized in that** the amount of peptide toxin and substance having an antagonistic effect and/or penetrant is selected depending on the tumor to be treated.

17. A pharmaceutical composition according to one or more of the preceding claims, **characterized in that** it contains conventional carriers and excipients and/or further active agents.

18. A pharmaceutical composition according to one or more of the preceding claims, **characterized in that** it contains as the conventional carriers and excipients isotonic solutions, protein solutions, amino acid solutions and/or biocidal solutions, preferably Ringer's solution, 0.9% NaCl solution, human albumine solution and/or glutamine solution, and that it contains as the further active agents antibiotics, antimycotics, antituberculosis agents, anti-parasite agents, amino acids, enzymes promoting wound-healing, mitosis inhibitors and/or cytostatics.

19. A pharmaceutical composition according to one or more of the preceding claims, **characterized in that** a derivative of said peptide toxin and/or said substance having an antagonistic effect and/or said penetrant is contained in the pharmaceutical preparation, and/or the peptide toxin and/or the substance having an antagonistic effect thereon and/or the penetrant are prepared by chemical synthesis or by recombinant biotechnological procedures and they correspond in their effect to the toxins or the substances having an antagonistic effect thereon and/or the penetrants and derivatives thereof contained in the poison of spiders of the family of Sicariidae.

20. A method for the preparation of a pharmaceutical composition comprising in a pharmaceutically effective amount at least a peptide toxin as well as at least a substance having an antagonistic effect thereon and/or at least a penetrant wherein the peptide toxin is derived from the poison of spiders of the family of Sicariidae and optionally the substance having an antagonistic effect thereon and/or the penetrant is derived from the poison of spiders of the family of Sicariidae, comprising the following steps of:
- preparing a raw spider poison mixture by procedures known per se as well as fractionating the mixture to obtain the peptide toxin and optionally the substance having an antagonistic effect thereon and/or the penetrant and optionally other substances in fractions which are separated from each other if possible;
- combining different fractions of the peptide toxin with fractions containing substances having an antagonistic effect thereon and/or penetrants, or with substances having an antagonistic effect and/or penetrants derived from other organisms to obtain a pharmaceutically effective composition.

21. A method according to claim 20, **characterized in that** the raw spider poison mixture is obtained from female spiders of the family of Sicariidae.

22. A method according to claim 20 and 21, **characterized in that** the raw spider poison mixture is obtained by manual milking.

23. A method according to one or more of claims 20-22, **characterized in that** the raw spider poison mixture is homogenized prior to fractionation.

24. A method according to one or more of the preceding claims 20 to 23, **characterized in that** prior to further processing into a pharmaceutical composition the fractions are deep-frozen and further preferably lyophilized.

25. Use of a pharmaceutical compositions according to one or more of claims 1-19 in medicine.

26. Use of a pharmaceutical compositions according to one or more of claims 1-19 in the treatment of tumor diseases and/or supportive treatment during tumor surgery.

## Revendications

1. Composition pharmaceutique pour le traitement d'affections tumorales, contenant en une quantité pharmaceutiquement efficace :
c) au moins une peptidotoxine, ainsi que
d) au moins une substance ayant un effet antagoniste vis-à-vis de celle-ci et/ou au moins une substance de pénétration,
dans laquelle au moins la peptidotoxine est issue du venin d'araignées de la famille des sicariidés et, au choix, la substance ayant un effet antagoniste vis-à-vis de celle-ci et/ou la substance de pénétration sont issues du venin d'araignées de la famille des sicariidés.

2. Composition pharmaceutique selon la revendication 1,
**caractérisée en ce qu'**elle contient une ou plusieurs autres substances issues du venin d'araignées de la famille des sicariidés.

3. Composition pharmaceutique selon la revendication 1 ou la revendication 2,
**caractérisée en ce que** la peptidotoxine et la substance ayant un effet antagoniste et/ou la substance de pénétration sont issues du venin des espèces d'araignées *Sicarius*, *Loxosceles, Scytodes* et/ou *Drymusa*.

4. Composition pharmaceutique selon l'une ou plusieurs des revendications précédentes,
**caractérisée en ce que** les araignées de l'espèce *Sicarius* sont les *Sicarius oweni, Sicarius testaceus, Sicarius hahni* et *Sicarius albospinosus*, les araignées de l'espèce *Loxosceles* sont les *Loxosceles rufescens, Loxosceles reclusa* et *Loxosceles laeta* et/ou les araignées de l'espèce *Scytodes* sont les *Scytodes thoracica, Scytodes rufa* et *Scytodes longipes*.

5. Composition pharmaceutique selon l'une ou plusieurs des revendications précédentes,
**caractérisée en ce que** la substance ayant un effet antagoniste et/ou l'enzyme de pénétration sont issues d'un autre organisme que les araignées de la famille des sicariidés.

6. Composition pharmaceutique selon l'une ou plusieurs des revendications précédentes,
**caractérisée en ce qu'**elle contient en plus en tant que peptidotoxine au moins un venin de serpent, de préférence du captopril du venin de la vipère cavernicole.

7. Composition pharmaceutique selon l'une ou plusieurs des revendications précédentes,
**caractérisée en ce qu'**elle contient en tant que substance à effet antagoniste et/ou que substance de pénétration au moins une hyaluronidase issue de venins de serpents, de préférence de venins de cobra, ou au moins une phospholipase issue d'*Actrataspis bibronii, Bitis arietans* ou *Vipera aspis zinnikeri.*

8. Composition pharmaceutique selon l'une ou plusieurs des revendications précédentes,
**caractérisée en ce que** la substance ayant un effet antagoniste et/ou la substance de pénétration est une phospholipase ou une hyaluronidase ou une combinaison de ces deux substances.

9. Composition pharmaceutique selon l'une ou plusieurs des revendications précédentes,
**caractérisée en ce que** la substance de pénétration est une phospholipase.

10. Composition pharmaceutique selon l'une ou plusieurs des revendications précédentes,
**caractérisée en ce que** la peptidotoxine et la substance ayant un effet antagoniste vis-à-vis de celle-ci et/ou la substance de pénétration ont été obtenues par un procédé de fractionnement à partir du venin d'araignée.

11. Composition pharmaceutique selon l'une ou plusieurs des revendications précédentes,
**caractérisée en ce que** la peptidotoxine et la substance ayant un effet antagoniste vis-à-vis de celle-ci et/ou la substance de pénétration a été obtenue par chromatographie sur gel, HPLC, chromatographie d'affinité et/ou chromatographie par échange d'ions à partir du venin d'araignée.

12. Composition pharmaceutique selon l'une ou plusieurs des revendications précédentes,
**caractérisée en ce que** la peptidotoxine et la substance ayant un effet antagoniste et/ou la substance de pénétration ont été obtenues comme suit :
en utilisant une colonne d'un diamètre intérieur de 1,5 cm et d'une hauteur de 50 cm, qui se rétrécit en forme de cône jusqu'à 1,5 mm et qui est remplie de 20 ml d'un gel chromatographique AcA 34, matrice : 3% d'acrylamide/4% d'agarose, plage de fractionnement : de 20 à 350 kDa, limite d'exclusion : 750 kDa, diamètre des sphérules 60 à 140 µm, du venin d'araignée issu de *Sicarius oweni*, *Sicarius testaceus, Sicarius hahni* ou *Sicarius albospinosus* dans du Tris/HCl 0,25 M pH 6,5 à 7,3 et de la glycine 1,92 M dans de l'eau distillée déminéralisée a été déposé dans un état homogène sur le gel et, lorsque la solution de venin a parcouru le gel et que 165 ml d'une solution de Tris/HCl 0,25 M pH 6,5 à 7,3 et de glycine 1,92 M dans de l'eau distillée déminéralisée ont été déposés, que les premiers 15 ml de l'éluat ont été jetés et que 4 ml de chacune des fractions ont été réunis, les peptidotoxines se trouvent dans les fractions 1, 2, 4, 7, 9 et 10 et les substances ayant un effet antagoniste et/ou les substances de pénétration se trouvent dans les fractions 3, 5, 6, 8, 11 et 12.

13. Composition pharmaceutique selon l'une ou plusieurs des revendications précédentes,
**caractérisée en ce qu'**elle contient une peptidotoxine et une substance ayant un effet antagoniste vis-à-vis de celle-ci et/ou une substance de pénétration qui sont issues de différentes fractions du venin d'araignée.

14. Composition pharmaceutique selon l'une ou plusieurs des revendications précédentes,
**caractérisée en ce que** la peptidotoxine et la substance ayant un effet antagoniste vis-à-vis de celle-ci et/ou la substance de pénétration sont présentes en une quantité de nature à produire vis-à-vis de cellules tumorales un effet de destruction de la peptidotoxine et de la substance ayant un effet antagoniste vis-à-vis de celle-ci et/ou de la substance de pénétration.

15. Composition pharmaceutique selon l'une ou plusieurs des revendications précédentes,
**caractérisée en ce que** la quantité est choisie de façon à assurer une distribution tissulaire contrôlée dans l'espace et/ou dans le temps.

16. Composition pharmaceutique selon l'une ou plusieurs des revendications précédentes,
**caractérisée en ce que** la quantité de peptidotoxine et de substance ayant un effet antagoniste et/ou de substance de pénétration est choisie en fonction de la tumeur à traiter.

17. Composition pharmaceutique selon l'une ou plusieurs des revendications précédentes,
**caractérisée en ce qu'**elle contient des véhicules et des adjuvants usuels et/ou d'autres principes actifs.

18. Composition pharmaceutique selon l'une ou plusieurs des revendications précédentes,
**caractérisée en ce qu'**elle contient en tant que véhicules et adjuvants usuels des solutions isotoniques, des solutions de protéines, des solutions d'acides aminés et/ou des solutions germicides, de préférence de la solution de Ringer, de la solution de NaCl à 0,9%, de la solution d'albumine humaine et/ou de la solution de glutamine, et **en ce qu'**elle contient comme autres principes actifs des antibiotiques, des antimycotiques, des antituberculeux, des agents de lutte contre les parasites, des acides aminés, des enzymes favorisant la cicatrisation des plaies, des inhibiteurs de la mitose et/ou des cytostatiques.

19. Composition pharmaceutique selon l'une ou plusieurs des revendications précédentes,
**caractérisée en ce qu'**un dérivé de la peptidotoxine et/ou de la substance ayant un effet antagoniste et/ou de la substance de pénétration est contenu dans la préparation pharmaceutique et/ou **en ce que** la peptidotoxine et/ou la substance ayant un effet antagoniste vis-à-vis de celle-ci et/ou la substance de pénétration sont produites par synthèse chimique ou par des méthodes biotechnologiques recombinantes et qu'elles correspondent du point de vue leur action aux toxines contenues dans le venin d'araignées de la famille des sicariidés ou à des substances ayant un effet antagoniste vis-à-vis de ces toxines et/ou à des substances de pénétration, et leurs dérivés.

20. Procédé de préparation d'une composition pharmaceutique contenant en une quantité pharmaceutiquement efficace au moins une peptidotoxine ainsi qu'au moins une substance ayant un effet antagoniste vis-à-vis de celle-ci et/ou au moins une substance de pénétration, dans laquelle la peptidotoxine est issue du venin d'araignées de la famille des sicariidés et, au choix, la substance ayant un effet antagoniste vis-à-vis de celle-ci et/ou la substance de pénétration sont issues du venin d'araignées de la famille des sicariidés, ledit procédé comprenant les étapes suivantes :
- Obtention d'un mélange brut de venin d'araignée par des procédés en soi connus et fractionnement du mélange afin d'obtenir la peptidotoxine et au choix la substance ayant un effet antagoniste vis-à-vis de celle-ci et/ou la substance de pénétration et au choix d'autres substances dans des fractions séparées le plus possible les unes des autres ;
- Combinaison de différentes fractions de la peptidotoxine avec des fractions contenant des substances ayant un effet antagoniste vis-à-vis de celle-ci et/ou des substances de pénétration ou avec des substances ayant un effet antagoniste et/ou des substances de pénétration provenant d'autres organismes afin d'obtenir une préparation pharmaceutiquement efficace.

21. Procédé selon la revendication 20,
**caractérisé en ce que** le mélange brut de venin d'araignée est obtenu à partir d'araignées femelles de la famille des sicariidés.

22. Procédé selon les revendications 20 et 21,
**caractérisé en ce que** le mélange brut de venin d'araignée est obtenu par collecte manuelle.

23. Procédé selon l'une ou plusieurs des revendications 20 à 22,
**caractérisé en ce que** le mélange brut de venin d'araignée est homogénéisé avant le fractionnement.

24. Procédé selon l'une ou plusieurs des revendications 20 à 23,
**caractérisé en ce que** les fractions sont congelées, de façon plus préférentielle lyophilisées, avant leur transformation ultérieure en une préparation pharmaceutique.

25. Utilisation de préparations pharmaceutiques selon l'une ou plusieurs des revendications 1 à 19 en médecine.

26. Utilisation de préparations pharmaceutiques selon l'une ou plusieurs des revendications 1 à 19 pour le traitement d'affections tumorales et/ou pour un traitement supplémentaire lors d'opérations sur des tumeurs.
